# FASCICULE DE BREVET EUROPEEN

(11) **EP 0 515 510 B1**
(45) Date de publication et mention de la délivrance du brevet: **26.04.1995**
(21) Numéro de dépôt: 91904756.3
(22) Date de dépôt: 13.02.1991
(51) Int. Cl.: C07C 233/47, C07C 229/08, C07D 207/28, A61K 7/48, A61K 31/13, A61K 31/40, A61K 31/165

(54) **ESTERS D'AMINOACIDES DE L'HYDROQUINONE, LEUR PROCEDE DE PREPARATION ET COMPOSITIONS PHARMACEUTIQUES OU COSMETIQUES LES CONTENANT**
HYDROQUINONESTER VON AMINOSÄUREN, VERFAHREN ZU IHRER HERSTELLUNG UND SIE ENTHALTENDE PHARMAZEUTISCHE ODER KOSMETISCHE ZUSAMMENSETZUNGEN
HYDROQUINONE AMINO-ACID ESTERS, METHOD OF PREPARATION, AND PHARMACEUTICAL OR COSMETIC COMPOSITIONS CONTAINING THEM

(30) Priorité: 15.02.1990 FR 9001822
(43) Date de publication de la demande: 02.12.1992
(73) Titulaire: VIRBAC S.A., F-06516 Carros Cedex (FR)
(72) Inventeur: LARUELLE, Claude, F-062700 Villeneuve-Loubet (FR); RAYNIER, Bernard, F-06200 Nice (FR); DERRIEU, Guy, F-06800 Cagnes-sur-Mer (FR)
(74) Mandataire: Ores, Irène
(86) Numéro de dépôt international: FR9100114
(87) Numéro de publication internationale: WO9111996

(56) Documents cités:
- EP-A- 0 060 092
- EP-A- 0 227 531
- DE-A- 2 456 634
- FR-A- 2 192 795
- FR-A- 2 577 805
- Journal of Chromatography, vol. 405, 18.09.87, Elsevier Science Publishers B.V. (Amsterdam, NL), J.S. Bradshaw et al.:"Polysiloxanes containing thermally stable chiral amide side-chains for capillary gas and supercritical fluid chromatography", pp. 169-177
- Chemical Abstracts, vol. 84, no. 17, 26.04.76 (Colombus, Ohio, US), M.S. Matta et al.: "Acylation of subtilisin Carlsberg by phenyl esters", page 203, résumé 117698j & J. Biol. Chem., 1976, 251(4), 1006-8

## Description

La présente invention est relative à des esters d'aminoacides de l'hydroquinone, aux compositions pharmaceutiques ou cosmétiques les contenant, en particulier les compositions dermatologiques à activité dépigmentante, ainsi qu'à leurs applications pharmaceutiques, cosmétiques ou esthétiques.

La présente invention est également relative à la préparation de ces nouveaux esters de l'hydroquinone, à partir de dérivés d'aminoacides naturels.

Il est connu que l'hydroquinone et ses dérivés simples, tels que le monométhyl éther, le monobenzyl éther, bien qu'ils soient considérés à l'heure actuelle comme les agents parmi les moins irritants des agents inhibiteurs de la tyrosinase et présentent un rapport efficacité/tolérance optimal, provoquent néanmoins une irritation et une sensibilisation importante de la peau, incompatible avec une utilisation prolongée en tant que dépigmentant, tant en dermatologie qu'en cosmétologie.

En effet, des topiques dépigmentants doivent impérativement obéir à trois règles: avoir un effet dépigmentant sur les seules lésions à traiter, ne produire ni irritation, ni pigmentation secondaire post-inflammatoire et ne provoquer ni allergie ni effet dépigmentant systémique.

Certains produits de synthèse, corticoïdes ou mercuriels, sont actifs sur la mélanogénèse, mais présentent un certain nombre d'effets secondaires qui contreindiquent une utilisation locale répétée.

Pour limiter l'irritation de la peau, un certain nombre d'associations ont été proposées. On peut citer notamment l'association hydroquinone - vitamine A acide et un corticoïde, qui a été en pratique limitée, en raison de l'existence d'autres effets secondaires [J.Invest. Dermatol. 73(5) p.357, (1979)].

D'autre part, l'hydroquinone et ses dérivés simples ont l'inconvénient majeur d'être sensibles à l'oxydation, même en présence de stabilisants tels que l'acide ascorbique ou le sulfure de sodium [J. Invest. Dermatol. 62, p.436, (1974)], ceci entraînant d'une part le brunissement des préparations et d'autre part la génération d'entités particulièrement irritantes pour la peau, limitant ainsi l'application topique de l'hydroquinone et de ses dérivés simples précités.

Afin de pallier ces différents inconvénients, d'autres dérivés de l'hydroquinone ont été proposés. Le brevet français n° 2 577 805 au nom de SHISEIDO et la Demande de Brevet européen n° 60092 au nom de SUNSTAR KABUSHIKI KAISHA revendiquent respectivement des hétérosides et des esters carboxyliques de 1 à 20 atomes de carbone de l'hydroquinone.

Cependant tant les produits décrits dans le Brevet français n° 2 577 805 que dans la Demande de Brevet européen présentent un certain nombre d'inconvénients ; en effet, les produits décrits dans le Brevet français n° 2 577 805 ont un caractère exclusivement hydrophile, qui ne permet pas la pénétration vers les couches profondes de la peau où l'action du principe actif peut être prolongée et qui limite ainsi l'efficacité de ces produits ; les produits décrits dans la Demande de Brevet européen n° 60092 ont pour leur part un caractère essentiellement lipophile qui limite également l'efficacité de ces produits, dans la mesure où leur diffusion au contact des fluides biologiques est faible.

La présente invention s'est, en conséquence, donné pour but de pourvoir à des dérivés de l'hydroquinone qui répondent mieux aux besoins de la pratique que les dérivés de l'Art antérieur, notamment en ce qu'ils présentent une stabilité remarquable à la lumière, à la chaleur et à l'eau.

La présente invention a pour objet des esters d' aminoacides naturels de l'hydroquinone pouvant présenter un caractère amphiphile, et les formulations ou compositions les contenant.

Ces produits répondent à la formule générale suivante (I) : 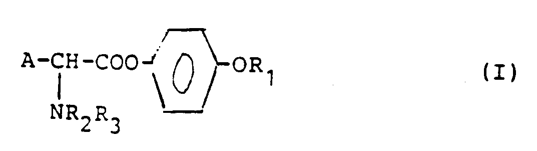
dans laquelle:
A correspond au reste d'un alpha aminoacide naturel ou d'un dérivé simple de ce dernier, tel un ester méthylique, éthylique, ou un amide primaire dans le cas des acides aminés dicarboxyliques ;
R₁ représente un atome d'hydrogène, un groupement alkyle comprenant 1 à 6 atomes de carbone ou un groupement benzyle ;
R₂ représente un atome d'hydrogène, un groupe acétyle ou benzoyle, un radical alkylecarbonyle avec 2 à 20 enchaînements hydrocarbonés ou bien définit avec A un cycle azoté,
à la condition que R₂ soit différent d'un atome d'hydrogène lorsque A et R₁ représentent des groupes méthyle, que R₂ soit différent d'un groupe benzoyle lorsque A représente un atome d'hydrogène et R₁ un groupe méthyle, et que R₂ soit différent d'un groupe acétyle lorsque A représente un groupe benzyle et R₁ représente un groupe méthyle ;
R₃ représente un atome d'hydrogène et, uniquement lorsque A et R₂ déterminent un cycle azoté, un radical alkylecarbonyle avec 1 à 20 enchaînements hydrocarbonés linéaires ou un radical alkyle linéaire ou ramifié avec 1 à 18 enchaînements hydrocarbonés.

Selon un mode de réalisation avantageux dudit dérivé, A représente un atome d'hydrogène, un cycle azoté en association avec R₂, ou un groupement alkyle comprenant 1 à 4 atomes de carbone, éventuellement substitué par l'un des groupes suivants, -OH, SH, C₆H₄-OH, CO-NH₂, COOH, NH₂, NH-CNH₂-NH₂, C₆H₅, indole, S-CH₃, imidazole lesquels substituants peuvent éventuellement être euxmême estérifiés.

A détermine ainsi le reste d'un alpha-aminoacide naturel choisi notamment parmi l'alanine, l'arginine, l'asparagine, l'acide aspartique, la cystéine, l'acide glutamique, l'acide pyroglutamique, la glutamine, la glycine, l'histidine, l'hydroxy-4 proline, l'isoleucine, la leucine, la lysine, la méthionine, l'ornithine, la phénylalanine, la proline, la sérine, la thréonine, le tryptophane, la tyrosine et la valine.

Par aminoacides naturels, on entend, au sens de la présente invention, les alpha aminoacides constitutifs des protéines. Le site chiral en position alpha détermine des isomères (l) et (d). La formule générale (I) inclut l'isomère (l) et la forme racémique de l'aminoacide. Le terme aminoacide comprend également, au sens de la présente invention, les dérivés les plus simples, comme les monoesters d'alkyle inférieurs dans le cas des acides aminés dicarboxyliques.

Au sens de la présente invention, le terme alkyle englobe notamment les radicaux méthyle, éthyle, propyle, butyle, pentyle, hexyle, heptyle, octyle, nonyle, décyle, undécyle, dodécyle, tridécyle, tetradécyle, pentadécyle, hexadécyle, heptadécyle, octadécyle, nonadécyle et éicosyle.

La Demanderesse a trouvé que, de façon surprenante, les produits répondant à la formule générale (I) présentent une activité dépigmentante au moins comparable et dans la plupart des cas supérieure à celle de l'hydroquinone et de ses dérivés simples, tout en ne présentant pas les inconvénients des produits de l'Art antérieur ; en effet, les produits de la présente invention ont, de manière inattendue, une stabilité remarquable à la lumière, à la chaleur, à l'eau, et peuvent être mélangés de façon appropriée avec toutes sortes de matières de base pharmaceutiques et cosmétiques hydrophiles et lipophiles.

Selon une disposition avantageuse de ce mode de réalisation, A détermine avec R₂ des produits comportant un cycle pyrrolidinone-2, lesquels produits répondent à la formule (II) suivante : 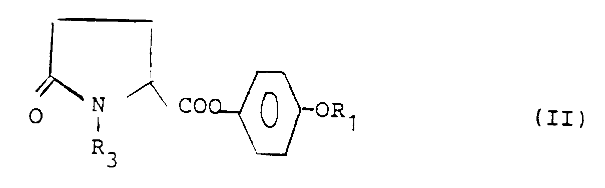
dans laquelle R₁ et R₃ ont les mêmes significations que précédemment.

Dans les produits de formule (II) selon l'invention, A définit ainsi avec R₂ un reste d'acide pyroglutamique.

Un certain nombre de documents décrivent l'intérêt du reste pyroglutamique dans le domaine dermatologique et cosmétique, et notamment le fait que le sel de sodium de l'acide (l) pyroglutamique prévient la déshydratation de la peau, en lui conférant douceur, souplesse et élasticité, (J. Soc. Cosmet. Chem. (1984) 35(3) 171) : le Brevet français n° 2 122 495 au nom de la société KARL THOMAE revendique l'emploi des pyroglutamates aliphatiques dans le traitement et l'hygiène de la peau ; UNILEVER, dans sa Demande de Brevet européen n° 176 217, propose l'emploi des esters de l'acide (l) pyroglutamique pour des préparations à usage topique, en particulier pour le traitement des coups de soleil, de l'acné, des hyperkeratoses ; MERCK, dans son brevet européen n° 227 531, revendique l'usage des pyroglutamates comme vecteurs exaltant la pénétration dermique des médicaments et la Société japonaise TEIJIN, dans sa Demande de Brevet européen n° 123 943 décrit des pyroglutamates de glycérol.

Cependant, les esters répondant à la formule (II) selon l'invention allient à la propriété inattendue de présenter une grande stabilité, une action prolongée sur l'humidité de la peau.

La Demanderesse pense que cette action inattendue est due au fait que les produits de formule (II) présentent une analogie avec les peptides naturels comportant un reste pyroglutamique, et que, de ce fait, ils sont des substrats de choix pour l'enzyme qui assure une étape de la synthèse de l'acide pyroglutamique présent dans le *stratum corneum*. Les esters de formule (II) conformes à l'invention, pénétrent aisément dans cette zone et contribuent à y assurer un taux conséquent d'acide pyroglutamique. Celui-ci, produit à l'intérieur des cellules du *stratum corneum*, est très peu sensible à l'élimination par lavages, de même que l'entité active libérée par hydrolyse. Celle-ci est donc à même d'avoir une action dépigmentante prolongée.

Les produits conformes à l'invention sont susceptibles d'être utilisés comme agents dépigmentants.

La présente invention a également pour objet l'utilisation et l'application de agents dépigmentants telles que définies aux revendications 10 et 11.

Les produits conformes à l'invention sont susceptibles d'être associés à des véhicules appropriés à la préparation de compositions pharmaceutiques et/ou cosmétiques, particulièrement efficaces dans le traitement des hyperpigmentations mélaniques indésirables et notamment les taches de rousseur (éphélides), le masque de grossesse (chloasma), les taches de vieillesse ou lentigo sénile, les hyperpigmentations post-traumatiques ou postlésionnelles, les réactions de photo-toxicité dues en particulier aux parfums.

Les mêmes compositions sont également particulièrement utiles pour les sujets qui souhaitent éclaircir leur teint jugé trop foncé.

La présente invention a également pour objet une méthode de traitement esthétique de l'Homme pour modifier la pigmentation de la peau, comprenant l'administration topique d'une quantité appropriée d'agent dépigmentant conforme à l'invention.

Les compositions pharmaceutiques et/ou cosmétiques selon la présente invention n'occasionnent pas d'irritation sensible à la peau et ne la sensibilisent pas, et peuvent, de ce fait, être appliquées en continu pendant une longue période de temps.

On peut utiliser indifféremment les composés de formule générale (I) dans les préparations appropriées pour l'application sur la peau, telle que pommades, crèmes, aérosols, poudres, teintures, gels, pâtes ou lotions. Les préparations correspondantes ont comme ingrédient actif un seul ou plusieurs des composés répondant à la formule générale (I), tel quel ou à l'état de sels pharmaceutiquement acceptables. Ces ingrédients actifs sont contenus habituellement à raison de 0,01 à 20 % en poids, de préférence de 0,1 à 10 % en poids. Il faut en effet noter que si la teneur en ingrédient actif dépasse 20 % aucune amélioration correspondante n'en découle, tandis que si celle-ci est inférieure à 0,01%, les effets envisagés ne sont plus garantis.

Les compositions selon la présente invention peuvent contenir tous les ingrédients classiques utilisés communément dans les compositions cosmétiques ou pharmaceutiques de traitement de la peau. Ces ingrédients sont par exemple :
. des huiles naturelles ou synthétiques, telles que paraffine liquide, huile de ricin, squalane, et huile de noix de coco ;
. des anti-oxydants tels que hydroxyanisole butylé, butylhydroxytoluène, gallate d'éthyle, et tocophérol ;
. des agents tensio-actifs tels que laurate de sodium, chlorure de laurylpyridinium, monooléate de polyoxyéthylène sorbitan, sodium N-stéaryl N,N-diméthylglycine, phosphate de glycéryléther de polyoxypropylène ;
. des humectants tels que glycérol, pyroglutamate de sodium, lactate de sodium ;
. des épaississants tels que gomme adragante, gomme de graine de coing, gomme de xanthane, carboxyvinylpolymère et bentonite ;
. des agents de conservation tels que acide benzoique, p-hydroxybenzoates d'alkyles, acide déhydroacétique et trichlorocarbanilide ;
. des agents colorants et des pigments tels que rouge acide, rhodamine B, violamine R, orange SS, naphtol, jaune-S, tartrazine, alizarine, vert de cyanine F, violet acide, carthamine, oxyde de fer bleu et jaune, dioxyde de titane, bleu de cobalt, bleu rose et violet d'outre-mer, noir de carbone ;
. des cires telles que cire d'abeille, cire du Japon, cire de carnauba, cire de candellila et lanoline ;
. des agents filmogènes tels que nitrocellulose et alcool polyvinylique ;
. des solvants tels que eau et alcools, par exemple éthanol
. des poudres telles que poudre d'aluminium, talc, kaolin, oxyde de zinc, dioxyde de titane, mica, carbonate de calcium ;
. des plastifiants tels que citrate d'acétyltributyle et dibutylphtalate ;
. des substances filtrantes des radiations UV, telles que celles du type acide p-aminobenzoïque et ester correspondant, du type salicylate, cinnamate, et dérivés de benzophénone ; et
. des parfums tels que musc, civette, ambre, jasmin et essence de rose.

Les composés répondant à la formule générale (I) 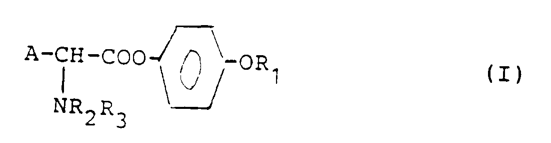
peuvent être préparés selon les techniques connues. Parmi celles-ci , on peut citer:
a) L'activation de la fonction acide carboxylique d'un aminoacide protégé à l'azote par un radical benzyloxycarbonyle et la réaction avec un dérivé p-OH-C₆H₄-OR₁, R₁ ayant ici comme dans ce qui suit la signification précédemment définie. Les réactifs activateurs de la fonction carboxylique sont choisis parmi le 1,1-carbonyldiimidazole, le 1,1-carbonyldi(1,2,4-triazole), le N-hydroxyphtalimide, le N-hydroxysuccinimide, de préférence le 1,1-carbonyldiimidazole. Le groupe protecteur est éliminé de façon douce par hydrogénolyse catalytique, menée dans un solvant inerte, alcool secondaire ou acide acétique, pour conduire aux composés répondant à la formule générale (III) : 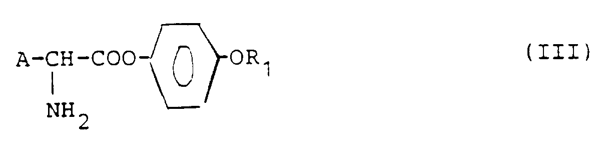
b) L'acylation d'un aminoacide en milieu biphasique eau/solvant organique par un dérivé réactif de l'acide R₂OH (R₂ représentant un radical alkylecarbonyle tel que précédemment défini), de préférence le chlorure correspondant. Le produit résultant peut être également préparé par acylation de l'ester benzylique de l'aminoacide correspondant par le même dérivé réactif, en milieu organique, avec accepteur d'acide, suivie d'une hydrogénolyse. Le produit résultant est activé avant réaction avec un dérivé p-OH-C₆H₄-OR₁, comme explicité au paragraphe a) pour conduire aux composés répondant à la formule (IV) : 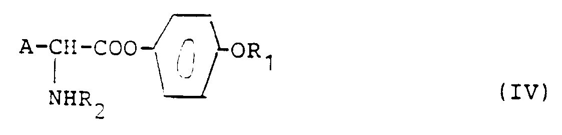
c) L'acylation de l'aminoacide correspondant, lorsque A et R₂ déterminent un cycle azoté, par un dérivé réactif de l'acide R₃OH (R₃ représentant un radical alkylecarbonyle tel que précédemment défini), selon les modalités mentionnées au paragraphe b).
   L'alkylation, -lorsque R₃ représente un radical alkyle tel que précédemment défini et A et R₂ déterminent un cycle azoté, -du sel sodique de l'ester benzylique de l'aminoacide correspondant est réalisée par un dérivé réactif R₃X, X étant un halogène, de préférence le brome. Cette alkylation est suivie d'une hydrogénolyse. Le sel sodique est généralement préparé *in situ* par l'intermédiaire d'hydrure ou de méthylate de sodium.
   Les produits obtenus, caractérisés par la formule (V) ci-après : 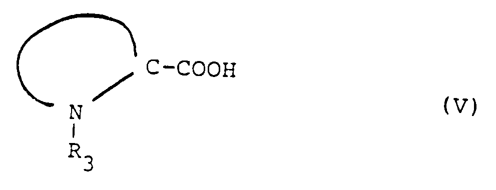 sont activés avant réaction avec un dérivé p-OH-C₆H₄-OR₁, comme explicité au paragraphe a), pour conduire aux composés répondant à la formule (II).
d) Enfin, pour obtenir les composés répondant à la formule (II), avec R₃ représentant l'hydrogène, on utilise une technique simple et performante qui consiste à effectuer une fusion directe, sans solvant et sous atmosphère inerte, de l'aminoacide correspondant avec le dérivé p-OH-C₆H₄-OR₁.

En général, les réactifs sont employés dans les rapports de la stoechiométrie ; les réactions sont menées dans des solvants inertes vis à des réactifs, tels que le chloroforme, le dichlorométhane, le dioxane, ou le diméthylformamide. Les accepteurs d'acide sont généralement des bases azotées tertiaires, de préférence la pyridine ou la triéthylamine.

Outre les dipositions qui précèdent, l'invention comprend encore d'autres dispositions, qui ressortiront de la description qui va suivre.

Les exemples qui suivent sont des illustrations des composés de formule (I), des diverses voies d'accès détaillées précédemment, des compositions dermatologiques ou cosmétiques les contenant et des tests d'évaluation de ces compositions.

Il doit être bien entendu, toutefois, que ces exemples de mise en oeuvre et de compte-rendu d'expérimentation sont donnés uniquement à titre d'illustration de l'objet de l'invention, dont ils ne constituent en aucune manière une limitation.

Tous les produits obtenus ont été soumis à l'analyse par chromatographie sur couche mince (CCM) et ne présentent qu'un seul spot. Les CCM ont été réalisées sur plaques Kieselgel F 254 et développées dans les systèmes suivants :
(i): toluène 10 ; formiate d'éthyle 10 ; acide formique 1 .
(ii): benzène 30 ; méthanol 1 .
(iii): n-butanol 8 ; acide acétique 1 ; eau 1

Les résultats des analyses centésimales pratiquées sur tous les produits sont conformes aux formules théoriques.

### EXEMPLE I : (L) PYROGLUTAMATE D'HYDROXY-4 PHENOL :

On mélange intimement 129 g (1 mole) d'acide (1) pyroglutamique et 110g (1 mole) d'hydroquinone, on agite à 175° sous courant d'azote pendant 18 heures, on revient à 50° pour diluer à l'eau (300 ml) et extraire à l'acétate d'éthyle (3 x 400 ml). On prépare ainsi un produit brut, purifié par deux empâtages à l'acétate d'éthyle (250 ml) pour conduire au dérivé du titre à l'état cristallisé, de pF= 179/82°. En CCM, le produit présente un seul spot, Rf(i) = 0,33.

### EXEMPLE II : N-HEXADECANOYL (L) ALANINATE D'HYDROXY-4 PHENOL :

### a): N-hexadecanoyl (1) alanine :

Le dérivé du titre est obtenu par acylation de la (l) alanine par une quantité stoechiométrique de chlorure de l'acide hexadécanoique, en milieu biphasique eau/éther éthylique, en présence de carbonate de sodium et de soude 10%. Il se présente à l'état cristallisé, pF= 94/7°, avec un seul spot en CCM, Rf(i) = 0.60 .

### b): N-hexadecanoyl (l) alaninate de benzyloxy-4 phénol :

On dissout 16.4 g (0.05 mole) de N-hexadecanoyl (l) alanine et 8.1 g (0.05 mole) de 1,1-carbonyldiimidazole, dans 100 ml de diméthylformamide, on agite à 5° pendant 1 heure, on coule 10 g (0.05 mole) de benzyloxy-4 phénol dans 50 ml de diméthylformamide, agite pendant 18 heures, on évapore puis on reprend au chloroforme. Aprés lavages à l'eau et à la soude diluée, et séchage, on obtient le dérivé du titre à l'état pur, pF= 77/9°, ne présentant qu'un seul spot en CCM, Rf(i)= 0.76.

### c): N-hexadecanoyl (l) alaninate d'hydroxy-4 phénol :

On soumet à l'hydrogénolyse le produit obtenu dans l'acide acétique, en présence de palladium sur charbon. En fin de réaction, on filtre, on évapore et on récupère le résidu dans l'éther éthylique, pour obtenir le dérivé du titre à l'état cristallisé, pF= 66/7°, ne présentant qu'un seul spot en CCM, Rf(i)= 0.49.

### EXEMPLES III à XV :

Selon les conditions explicitées précédemment, on prépare les produits suivants :
III : N-benzoyl glycinate de benzyloxy-4 phénol : pF= 138/40°.
IV : N-benzoyl glycinate d'hydroxy-4 phénol : pF= 149/51°
V : N-hexadecanoyl (l) alaninate de méthoxy-4 phénol : pF= 70/1°
VI : N-octyl (l) pyroglutamate de méthoxy-4 phénol : pF= 95/7°
VII : N-hexanoyl (l) pyroglutamate de méthoxy-4 phénol : pF= 103/5°
VIII : (l) alaninate d'hydroxy-4 phénol : pF= 154/8°
IX : N-décanoyl (l) valinate de benzyloxy -4 phénol : pF= 88/9°
X : N-décanoyl (l) valinate d'hydroxy-4 phénol : pF= 64/6°
XI : N-décanoyl (l) valinate de méthoxy-4 phénol : pF= 81/3°
XII : N-dodecanoyl (l) leucinate de benzyloxy-4 phénol : pF=80/2°
XIII : N-dodecanoyl (l) leucinate d'hydroxy-4 phénol : pF= 61/3°
XIV : N-octadecanoyl (l) valinate de méthoxy-4 phénol : pF= 66/8°
XV : N-hexanoyl, γ méthyl , (l) glutamate d'hydroxy-4 phénol : pF= 102/5°

### EXEMPLE XVI : COMPOSITION DEPIGMENTANTE n°1 :

| Ingrédients | % |
|---|---|
| Acide stéarique : | 6 |
| Alcool stéarylique | 5 |
| Stéarate de butyle | 8 |
| Monostéarate de glycérol | 2 |
| Cinnamate d'octyle | 2 |
| Propyleneglycol | 10 |
| Produit selon l'exemple X : | 6,5 |
| Hydroxyde de potassium : | 0,2 |
| p-Hydroxybenzoate de méthyle : | 0,09 |
| p-Hydroxybenzoate de propyle : | 0,01 |
| Butyl hydroxy toluène : | 0,02 |
| Eau déminéralisée : | qsp 100 |

Le propylèneglycol et l'hydroxyde de potassium sont dissous dans l'eau et la phase aqueuse portée à une température de 70°. Les autres ingrédients sont mélangés ensemble, fondus par chauffage et maintenus à une température voisine de 70°. La phase huileuse est ajoutée à la phase aqueuse, et le mélange émulsionné avec un homogénéiseur. Le produit est refroidi vers la température ambiante sous agitation.

### EXEMPLE XVII : COMPOSITION DEPIGMENTANTE n°2

| Ingrédients | % |
|---|---|
| Cire d'abeille | 10 |
| Cérésine | 7 |
| Vaseline blanche | 3 |
| Lanoline hydratée | 2 |
| Myristate d'isopropyle | 2 |
| Squalane | 2 |
| Paraffine liquide | 40 |
| Ether cétylique de polyoxyéthylene | 2 |
| Produit selon l'exemple I | 4 |
| Monostéarate de glycérol | 2 |
| Propylèneglycol | 2 |
| Butyl hydroxy toluène | 0,02 |
| eau déminéralisée | qsp 100 |

La préparation s'éffectue selon l'exemple XVI

### EXEMPLE XVIII : COMPOSITION DEPIGMENTANTE n° 3

| | |
|---|---|
| Alcool éthylique (95%) | 30 |
| Dipropylèneglycol | 18 |
| Ether oleïque de polyoxyéthylène | 2,5 |
| Polymère carboxyvinylique | 1,5 |
| Hydroxyde de potassium | 0,2 |
| (l) Arginine | 0,15 |
| Produit selon l'exemple XIII | 7,5 |
| p-Hydroxybenzoate de méthyle | 0,09 |
| p-Hydroxybenzoate de propyle | 0,01 |
| Eau déminéralisée | qsp 100 |

On dissout le polymère carboxyvinylique dans l'eau, et le dipropylèneglycol, l'éther oléique, le produit selon l'exemple XIII, les conservateurs sont dissous dans l'éthanol. La solution alcoolique est ajoutée à la solution aqueuse, la solution résultante est neutralisée et épaissie avec l'hydroxyde de potassium et la (l) arginine .

### EXEMPLE XIX: RESULTATS DES TESTS D'EVALUATION DES COMPOSITIONS SELON LES EXEMPLES XVI à XVIII :

Les compositions correspondantes présentent une teneur potentielle en hydroquinone de 2% . Elles sont comparées à une composition n°4, préparée selon l'exemple XVI mais contenant 2% d'hydroquinone à la place du produit selon l'exemple X.

### a) Activité dépigmentante :

Elle est évaluée par application des échantillons à 20 sujets souffrant de chloasma. Chacun des sujets applique matin et soir sur 4 zones différentes les 4 compositions, à raison de 0,2 g chaque fois, chaque zone recevant le même produit pendant toute la durée du traitement, soit 8 semaines.

Les améliorations sont observées visuellement chaque semaine, l'expérimentateur appréciant également la tolérance au traitement en notant toutes manifestations cutanées ou autres pouvant avoir pour origine le traitement en cours. On a pu noter ainsi l'intêret particulier des compositions selon l'invention, parfaitement tolérées et présentant une activité dépigmentante trés significative.

Le Tableau I ci-dessous présente les résultats obtenus à l'issue du traitement.

**Tableau I**

| Notation (*) | Composition n°1 | Composition n°2 | Composition n°3 | Composition n°4 |
|---|---|---|---|---|
| xxx | 4 | 4 | 3 | 2 |
| xx | 11 | 9 | 10 | 8 |
| x | 5 | 8 | 6 | 8 |
| o | 0 | 1 | 1 | 2 |

| | | | | |
|---|---|---|---|---|
| (*) xxx : amélioration remarquable xx : amélioration sensible x : légère amélioration o : pas d'amélioration | | | | |

### b) Stabilité :

On observe le changement de coloration des compositions 1 et 4 en stockant les échantillons à 37° pendant 12 semaines. Les modifications sont visuellement évaluées et représentées dans le Tableau II ci-après, confirmant la stabilité remarquable de la composition contenant un composé conforme à l'invention.

**Tableau II**

| Composition | Durée de stockage (semaines) | | | | |
|---|---|---|---|---|---|
| | 1 | 3 | 6 | 9 | 12 |
| 1 | o | o | o | o | o |
| 4 | x | xx | xxx | xxx | xxx |
| (*) o : pas de changement x : brunissement léger xx : brunissement sensible xxx : brunissement considérable | | | | | |

### EXEMPLE XX : APPRECIATION DE LA TOLERANCE SUPERFICIELLE ET TRANSCUTANEE PAR APPLICATIONS ITERATIVES.

On a comparé les compositions n° 2 et n° 4, la composition n° 5, préparée selon l'exemple XVII avec 4 % de monohexanoate d'hydroquinone à la place du produit selon l'exemple I, la composition n° 6 préparée selon l'exemple XVIII, avec 5 % d'arbutine à la place du produit selon l'exemple XIII.

Des applications cutanées quotidiennes, pendant 42 jours consécutifs, sur 24 lapins tondus régulièrement tous les 7 jours, à raison de 6 lapins par composition, sont réalisées au niveau de la région dorsale rétroscapulaire, à raison de 2 g de produit que l'on fait pénétrer par un massage doux.

On apprécie préférentiellement les érythèmes et les formations d'oedèmes, selon l'échelle numérique suivante :
- pas d'érythème (ou pas d'oedème) : 0
- léger érythème (ou très léger oedème) à peine visible: 1
- érythème bien visible (ou léger oedème) : 2
- érythème important (ou oedème important) : 3

Les résultats sont consignés dans le Tableau III, où apparaît le nombre de lapins correspondant à chacune des évaluations.

**Tableau III**

| N° COMPOSITION | DUREE (semaines) | ERYTHEME | | | | OEDEME | | | |
|---|---|---|---|---|---|---|---|---|---|
| | | 0 | 1 | 2 | 3 | 0 | 1 | 2 | 3 |
| 2 | 2 | 6 | | | | 6 | | | |
| | 6 | 5 | 1 | | | 6 | | | |
| 4 | 2 | | 1 | 3 | 2 | | 2 | 3 | 1 |
| | 6 | | | 2 | 4 | | | 2 | 4 |
| 5 | 2 | 1 | 2 | 3 | | 2 | 4 | | |
| | 6 | | 2 | 3 | 1 | 1 | 3 | 2 | |
| 6 | 2 | 6 | | | | 5 | 1 | | |
| | 6 | 4 | 2 | | | 4 | 2 | | |

Au cours de cette étude, ont été également appréciés, une fois par semaine, l'aspect et la souplesse de la peau, la vitesse et l'aspect de l'empoilement, l'épaisseur du pli cutané.

Toutes ces observations, ainsi que les résultats figurant dans le Tableau III, précisent l'intérêt particulier des produits selon l'invention par rapport aux autres agents de dépigmentation.

### EXEMPLE XXI : APPRECIATION DE LA RETENTION DANS LES TISSUS SOUS-CUTANES.

On compare les compositions n° 3 et n° 6, sur six rats, à raison de trois rats par composition, en administration unique de 1 g/rat, sur une zone dorsale préalablement rasée et parfaitement délimitée. Après 8 heures, la zone exposée est lavée avec un coton imbibé d'alcool, les animaux sacrifiés, les tissus correspondant au derme, épiderme et stratum corneum excisés, homogénéisés, traités et extraits pour dosage HPLC, en équivalent du principe actif (P.A.) engagé. Les résultats sont rapportés dans le Tableau IV.

**Tableau IV**

| N° COMPOSITION | DOSE g/rat | DOSE P.A. mg | Qté Eq. PA (a) accumulée |
|---|---|---|---|
| 3 | 1 | 40 | 2,5 (± 0,85) |
| 6 | 1 | 50 | 0,45 (± 0,12) |

| | | | |
|---|---|---|---|
| (a) : expression en mg/g de tissu | | | |

Ces résultats, joints aux éléments consignés dans les exemples XIX et XX, confirment l'intérêt particulier des produits selon l'invention par rapport aux glucosides d'hydroquinone.

## Revendications (Revendications pour l'(les) Etat(s) contractant(s) suivant(s): AT, BE, CH, LI, DE, DK, FR, GB, IT, LU, NL, SE)

1. Dérivé de l'hydroquinone, caractérisé en ce qu'il est constitué par à un ester d'aminoacide naturel de l'hydroquinone et en ce qu'il répond à la formule suivante (I) : 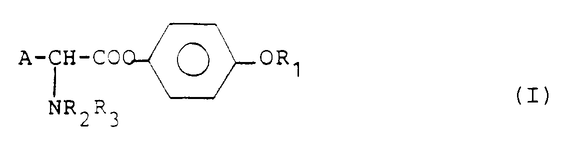 dans laquelle:
A correspond au reste d'un alpha aminoacide naturel ou d'un dérivé simple de ce dernier, tel un ester méthylique, éthylique, ou un amide primaire dans le cas des acides aminés dicarboxyliques ;
R₁ représente un atome d'hydrogène, un groupement alkyle comprenant 1 à 6 atomes de carbone ou un groupement benzyle ;
R₂ représente un atome d'hydrogène, un groupe acétyle ou benzoyle, un radical alkylecarbonyle avec 2 à 20 enchaînements hydrocarbonés ou bien définit avec A un cycle azoté,
à la condition que R₂ soit différent d'un atome d'hydrogène lorsque A et R₁ représentent des groupes méthyle, que R₂ soit différent d'un groupe benzoyle lorsque A représente un atome d'hydrogène et R₁ un groupe méthyle, et que R₂ soit différent d'un groupe acétyle lorsque A représente un groupe benzyle et R₁ un groupe méthyle ;
R₃ représente un atome d'hydrogène et, uniquement lorsque A et R₂ déterminent un cycle azoté, un radical alkylecarbonyle avec 1 à 20 enchaînements hydrocarbonés linéaires ou un radical alkyle linéaire ou ramifié avec 1 à 18 enchaînements hydrocarbonés.

2. Dérivé selon la revendication 1, caractérisé en ce que A représente un atome d'hydrogène, un cycle azoté en association avec R₂, ou un groupement alkyle comprenant 1 à 4 atomes de carbone, éventuellement substitué par l'un des groupes suivants, OH, SH, C₆H₄-OH, CO-NH₂, COOH, NH₂, NH-CNH₂-NH₂, C₆H₅, indole, S-CH₃, imidazole lesquels substituants peuvent éventuellement être eux-même estérifiés.

3. Dérivé selon la revendication 1 ou la revendication 2, caractérisé en ce que A détermine avec R₂ des produits comportant un cycle pyrrolidinone-2, A définissant ainsi avec R₂ un reste d'acide pyroglutamique, lesquels produits répondent à la formule (II) suivante : 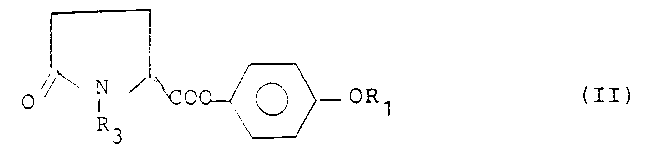 dans laquelle R₁ et R₃ ont les mêmes significations que précédemment.

4. Dérivé selon l'une quelconque des revendications 1 à 3, caractérisé en ce qu'il présente un caractère amphiphile.

5. Dérivé selon l'une quelconque des revendications 1, 2 ou 4, caractérisé en ce que le radical R₂ représente un radical alkylecarbonyle.

6. Dérivé selon l'une quelconque des revendications 1 à 4, caractérisé en ce que le radical R₃ représente un groupe alkyle.

7. Dérivé selon l'une quelconque des revendications 1 à 4, caractérisé en ce que le radical R₃ représente un radical alkylecarbonyle.

8. Composition pharmaceutique, caractérisée en ce qu'elle comprend au moins un dérivé de l'hydroquinone selon l'une quelconque des revendications 1 à 7, associé à au moins un véhicule pharmaceutiquement acceptable.

9. Composition pharmaceutique, utile en tant qu'agent dépigmentant, caractérisée en ce qu'elle comprend au moins un dérivé de l'hydroquinone selon l'une quelconque des revendications 1 à 7.

10. Utilisation d'un dérivé de l'hydroquinone de formule I selon la revendication 1, qui répond à la formule I' : 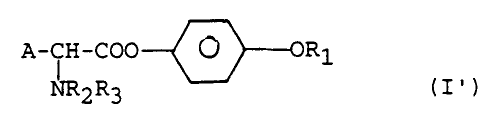 dans laquelle A, R₁ et R₃ ont la même signification que précédemment et dans laquelle
- si R₂ est un atome d'hydrogène, A et R₁ représentent des groupes méthyle, ou
- si R₂ est un groupe benzoyle, A représente un atome d'hydrogène et R₁ un groupe méthyle, ou
- si R₂ est un groupe acétyle, A représente un groupe benzyle et R₁ un groupe méthyle,
pour l'obtention d'un médicament destiné à une utilisation dépigmentante.

11. Application à titre de produit cosmétique d'un dérivé de l'hydroquinone de formule I selon la revendication 1, qui répond à la formule I' : 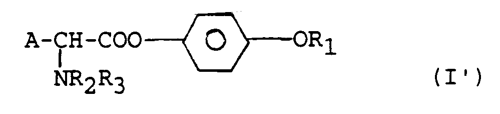 dans laquelle A, R₁ et R₃ ont la même signification que précédemment et dans laquelle
- si R₂ est un atome d'hydrogène, A et R₁ représentent des groupes méthyle, ou
- si R₂ est un groupe benzoyle, A représente un atome d'hydrogène et R₁ un groupe méthyle, ou
- si R₂ est un groupe acétyle, A représente un groupe benzyle et R₁ un groupe méthyle.

12. Composition cosmétique, caractérisée en ce qu'elle renferme au moins un produit de formule I selon l'une quelconque des revendications 1 à 7, éventuellement associé à au moins un véhicule acceptable.

13. Composition selon l'une quelconque des revendications 8, 9 ou 12, caractérisée en ce qu'elle comprend de 0,01 à 20 % en poids d'un produit de formule I selon l'une quelconque des revendications 1 à 7.

14. Composition selon la revendication 13, caractérisée en ce qu'elle contient de 0,1 à 10% en poids d'un produit de formule I selon l'une quelconque des revendications 1 à 7, éventuellement associé à au moins un véhicule acceptable.

15. Méthode de traitement esthétique de l'Homme pour modifier la pigmentation de la peau, comprenant l'administration topique d'une quantité appropriée d'un produit choisi dans le groupe qui comprend les produits de formule I selon l'une quelconque des revendications 1 à 7 et les produits de formule I' : 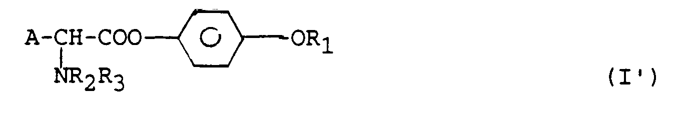 dans laquelle A, R₁ et R₃ ont la même signification que précédemment et dans laquelle
- si R₂ est un atome d'hydrogène, A et R₁ représentent des groupes méthyle, ou
- si R₂ est un groupe benzoyle, A représente un atome d'hydrogène et R₁ un groupe méthyle, ou
- si R₂ est un groupe acétyle, A représente un groupe benzyle et R₁ un groupe méthyle,
éventuellement associé à au moins un véhicule acceptable.

16. Méthode selon la revendication 15, caractérisé en ce que le produit de formule I ou le produit de formule I' est sous la forme d'une composition comprenant 0,01 à 20 % en poids dudit produit.

17. Méthode selon la revendication 16, caractérisée en ce que lesdits produits sont sous la forme d'une composition comprenant 0,1 à 10 % en poids desdits produits.

18. Procédé de préparation des dérivés de formule (I) selon la revendication 1, caractérisé en ce que lorsque R₂ et R₃ représentent un atome d'hydrogène, l'on fait réagir un dérivé de formule p-OH-C₆H₄-OR₁, dans laquelle R₁ a la même signification que précédemment avec un aminoacide approprié protégé à l'azote et dont la fonction acide carboxylique est activée par un réactif activateur convenable, puis en ce que l'on élimine ledit groupement protecteur par hydrogénolyse catalytique, menée dans un solvant inerte approprié, pour conduire aux composés répondant à la formule (III) ci-après :

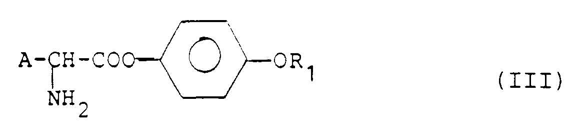

19. Procédé selon la revendication 18, caractérisé en ce que les réactifs activateurs de la fonction carboxylique sont choisis parmi le 1,1-carbonyldiimidazole, le 1,1-carbonyldi(1,2,4-triazole), le N-hydroxyphtalimide, le N-hydroxysuccinimide, de préférence le 1,1-carbonyldiimidazole.

20. Procédé de préparation des dérivés selon la revendication 5, caractérisé en ce que l'on réalise l'acylation d'un aminoacide approprié en milieu biphasique eau/solvant organique, par un dérivé réactif de l'acide R₂OH (R₂ représentant un radical alkylecarbonyle tel que précédemment défini), de préférence le chlorure correspondant pour conduire aux composés répondant à la formule (IV) :

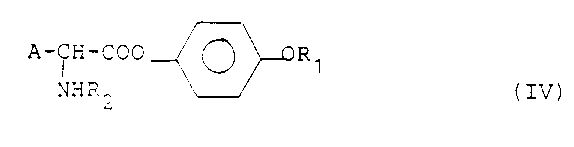

21. Procédé de préparation des dérivés de formule (II), caractérisé en ce que lorsque R₃ représente un atome d'hydrogène on effectue une fusion directe, sans solvant et sous atmosphère inerte, de l'aminoacide correspondant avec le dérivé p-OH-C₆H₄-OR₁, R₁ ayant la même signification que ci-dessus.

22. Produit intermédiaire pour la préparation des dérivés de formule I selon la revendication 1, caractérisé en ce qu'il répond à la formule III suivante : 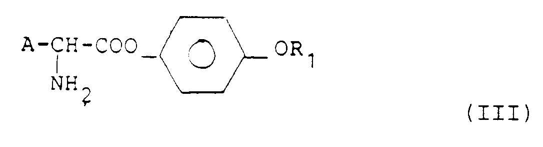 dans laquelle
A correspond au reste d'un alpha aminoacide naturel ou d'un dérivé simple de ce dernier, tel un ester méthylique, éthylique, ou un amide primaire dans le cas des acides aminés dicarboxyliques ;
R₁ représente un atome d'hydrogène, un groupement alkyle comprenant 1 à 6 atomes de carbone ou un groupement benzyle.

23. Produit intermédiaire pour la préparation des dérivés selon la revendication 5, caractérisé en ce qu'il répond à la formule IV suivante : 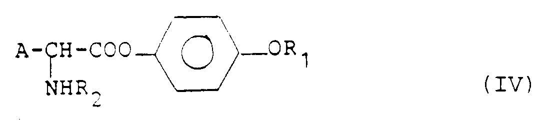 dans laquelle
A correspond au reste d'un alpha aminoacide naturel ou d'un dérivé simple de ce dernier, tel un ester méthylique, éthylique, ou un amide primaire dans le cas des acides aminés dicarboxyliques ;
R₁ représente un atome d'hydrogène, un groupement alkyle comprenant 1 à 6 atomes de carbone ou un groupement benzyle ;
et R₂ a la même signification qu'à la revendication 5, à la condition que R₂ soit différent d'un groupe acétyle lorsque A représente un groupe benzyle et R₁ un groupe méthyle.

## Revendications (Revendications pour l'(les) Etat(s) contractant(s) suivant(s): ES, GR)

1. Procédé d'obtention d'un dérivé de l'hydroquinone, caractérisé en ce qu'il comprend pour l'obtention d'un ester d'aminoacide naturel de l'hydroquinone répondant à la formule suivante (I) : 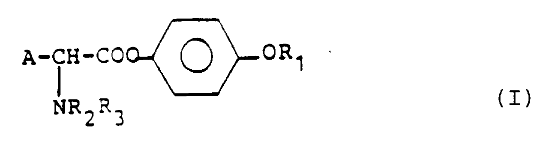 dans laquelle:
A correspond au reste d'un alpha aminoacide naturel ou d'un dérivé simple de ce dernier, tel un ester méthylique, éthylique, ou un amide primaire dans le cas des acides aminés dicarboxyliques ;
R₁ représente un atome d'hydrogène, un groupement alkyle comprenant 1 à 6 atomes de carbone ou un groupement benzyle ;
R₂ représente un atome d'hydrogène, un groupe acétyle ou benzoyle, un radical alkylecarbonyle avec 2 à 20 enchaînements hydrocarbonés ou bien définit avec A un cycle azoté,
à la condition que R₂ soit différent d'un atome d'hydrogène lorsque A et R₁ représentent des groupes méthyle, que R₂ soit différent d'un groupe benzoyle lorsque A représente un atome d'hydrogène et R₁ un groupe méthyle, et que R₂ soit différent d'un groupe acétyle lorsque A représente un groupe benzyle et R₁ un groupe méthyle ;
R₃ représente un atome d'hydrogène et, uniquement lorsque A et R₂ déterminent un cycle azoté, un radical alkylecarbonyle avec 1 à 20 enchaînements hydrocarbonés linéaires ou un radical alkyle linéaire ou ramifié avec 1 à 18 enchaînements hydrocarbonés,
la réaction d'un aminoacide et d'un dérivé p-OH-C₆H₄-OR₁ dans lequel R₁ à la même signification que ci-dessus.

2. Procédé d'obtention d'un dérivé selon la revendication 1, caractérisé en ce que A représente un atome d'hydrogène, un cycle azoté en association avec R₂, ou un groupement alkyle comprenant 1 à 4 atomes de carbone, éventuellement substitué par l'un des groupes suivants, OH, SH, C₆H₄-OH, CO-NH₂, COOH, NH₂, NH-CNH₂-NH₂, C₆H₅, indole, S-CH₃, imidazole lesquels substituants peuvent éventuellement être eux-même estérifiés.

3. Procédé d'obtention d'un dérivé selon la revendication 1 ou la revendication 2, caractérisé en ce que A détermine avec R₂ des produits comportant un cycle pyrrolidinone-2, A définissant ainsi avec R₂ un reste d'acide pyroglutamique, lesquels produits répondent a la formule (II) suivante : 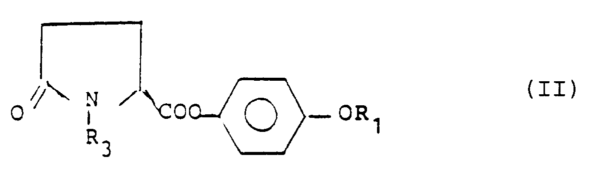 dans laquelle R₁ et R₃ ont les mêmes significations que précédemment.

4. Procédé d'obtention d'un dérivé selon l'une quelconque des revendications 1 à 3, caractérisé en ce que le produit obtenu présente un caractère amphiphile.

5. Procédé d'obtention d'un dérivé selon l'une quelconque des revendications 1, 2 ou 4, caractérisé en ce que le radical R₂ représente un radical alkylecarbonyle.

6. Procédé d'obtention d'un dérivé selon l'une quelconque des revendications 1 à 4, caractérisé en ce que le radical R₃ représente un groupe alkyle.

7. Procédé d'obtention d'un dérivé selon l'une quelconque des revendications 1 à 4, caractérisé en ce que le radical R₃ représente un radical alkylecarbonyle.

8. Procédé d'obtention d'une composition pharmaceutique, caractérisé en ce qu'il comprend l'incorporation à ladite composition d'au moins un dérivé de l'hydroquinone obtenu selon l'une quelconque des revendications 1 à 7, et d'au moins un véhicule pharmaceutiquement acceptable.

9. Procédé d'obtention d'une composition pharmaceutique, utile en tant qu'agent dépigmentant, caractérisé en ce qu'il comprend l'incorporation à ladite composition d'au moins un dérivé de l'hydroquinone obtenu selon l'une quelconque des revendications 1 à 7.

10. Procédé d'obtention d'un médicament comprenant la préparation d'un dérivé de l'hydroquinone de formule I obtenu selon la revendication 1, qui répond à la formule I' : 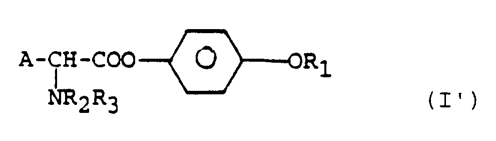 dans laquelle A, R₁, R₂ et R₃ ont la même signification que précédemment et dans laquelle
- si R₂ est un atome d'hydrogène, A et R₁ représentent des groupes méthyle, ou
- si R₂ est un groupe benzoyle, A représente un atome d'hydrogène et R₁ un groupe méthyle, ou
- si R₂ est un groupe acétyle, A représente un groupe benzyle et R₁ un groupe méthyle,
caractérisé en ce que le médicament obtenu est destiné à une utilisation dépigmentante.

11. Méthode de traitement cosmétique comprenant l'administration d'un dérivé de l'hydroquinone de formule I obtenu selon la revendication 1, qui répond à la formule I' : 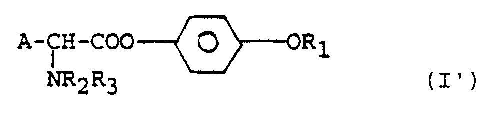 dans laquelle A, R₁, R₂ et R₃ ont la même signification que précédemment et dans laquelle
- si R₂ est un atome d'hydrogène, A et R₁ représentent des groupes méthyle, ou
- si R₂ est un groupe benzoyle, A représente un atome d'hydrogène et R₁ un groupe méthyle, ou
- si R₂ est un groupe acétyle, A représente un groupe benzyle et R₁ un groupe méthyle.

12. Procédé d'obtention d'une composition cosmétique, caractérisé en ce qu'il comprend l'incorporation à ladite composition d'au moins un produit de formule I obtenu selon l'une quelconque des revendications 1 à 7, et éventuellement d'au moins un véhicule acceptable.

13. Procédé d'obtention d'une composition obtenue selon l'une quelconque des revendications 8, 9 ou 12, caractérisé en ce qu'il comprend l'incorporation à ladite composition de 0,01 à 20 % en poids d'un produit de formule I obtenu selon l'une quelconque des revendications 1 à 7.

14. Procédé d'obtention d'une composition selon la revendication 13, caractérisé en ce qu'il comprend l'incorporation à ladite composition de 0,1 à 10% en poids d'un produit de formule I obtenu selon l'une quelconque des revendications 1 à 7, et éventuellement l'incorporation d'au moins un véhicule acceptable.

15. Méthode de traitement esthétique de l'Homme pour modifier la pigmentation de la peau, comprenant l'administration topique d'une quantité appropriée d'un produit choisi dans le groupe qui comprend les produits de formule I selon l'une quelconque des revendications 1 à 7 et les produits de formule I' : 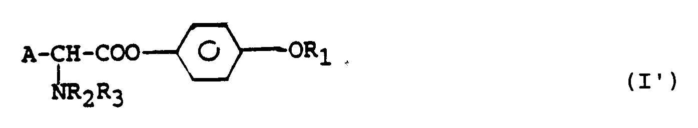 dans laquelle A, R₁, R₂ et R₃ ont la même signification que précédemment et dans laquelle
- si R₂ est un atome d'hydrogène, A et R₁ représentent des groupes méthyle, ou
- si R₂ est un groupe benzoyle, A représente un atome d'hydrogène et R₁ un groupe méthyle, ou
- si R₂ est un groupe acétyle, A représente un groupe benzyle et R₁ un groupe méthyle,
éventuellement associé à au moins un véhicule acceptable.

16. Méthode selon la revendication 15, caractérisé en ce que le produit de formule I ou le produit de formule I' est sous la forme d'une composition comprenant 0,01 à 20 % en poids dudit produit.

17. Méthode selon la revendication 16, caractérisée en ce que lesdits produits sont sous la forme d'une composition comprenant 0,1 à 10 % en poids desdits produits.

18. Procédé de préparation des dérivés de formule (I) selon la revendication 1, caractérisé en ce que lorsque R₂ et R₃ représentent un atome d'hydrogène, l'on fait réagir un dérivé de formule p-OH-C₆H₄-OR₁, dans laquelle R₁ a la même signification que précédemment avec un aminoacide approprié protégé à l'azote et dont la fonction acide carboxylique est activée par un réactif activateur convenable, puis en ce que l'on élimine ledit groupement protecteur par hydrogénolyse catalytique, menée dans un solvant inerte approprié, pour conduire aux composés répondant à la formule (III) ci-après :

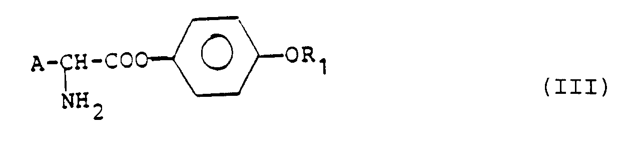

19. Procédé selon la revendication 18, caractérisé en ce que les réactifs activateurs de la fonction carboxylique sont choisis parmi le 1,1-carbonyldiimidazole, le 1,1-carbonyldi(1,2,4-triazole), le N-hydroxyphtalimide, le N-hydroxysuccinimide, de préférence le 1,1-carbonyldiimidazole.

20. Procédé de préparation des dérivés selon la revendication 5, caractérisé en ce que l'on réalise l'acylation d'un aminoacide approprié en milieu biphasique eau/solvant organique, par un dérivé réactif de l'acide R₂OH (R₂ représentant un radical alkylecarbonyle tel que précédemment défini), de préférence le chlorure correspondant pour conduire aux composés répondant à la formule (IV) :

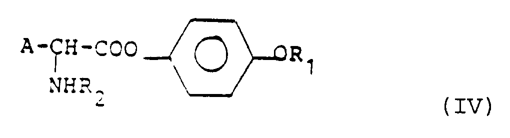

21. Procédé de préparation des dérivés de formule (II), caractérisé en ce que lorsque R₃ représente un atome d'hydrogène on effectue une fusion directe, sans solvant et sous atmosphère inerte, de l'aminoacide correspondant avec le dérivé p-OH-C₆H₄-OR₁, R₁ ayant la même signification que ci-dessus.

22. Produit intermédiaire pour la préparation des dérivés de formule I selon la revendication 1, caractérisé en ce qu'il répond à la formule III suivante : 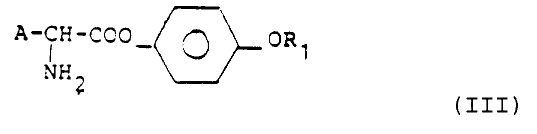 dans laquelle
A correspond au reste d'un alpha aminoacide naturel ou d'un dérivé simple de ce dernier, tel un ester méthylique, éthylique, ou un amide primaire dans le cas des acides aminés dicarboxyliques ;
R₁ représente un atome d'hydrogène, un groupement alkyle comprenant 1 à 6 atomes de carbone ou un groupement benzyle.

23. Produit intermédiaire pour la préparation des dérivés selon la revendication 5, caractérisé en ce qu'il répond à la formule IV suivante : 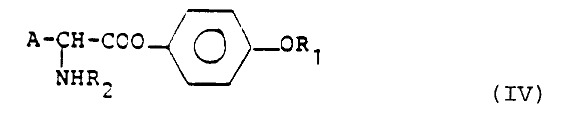 dans laquelle
A correspond au reste d'un alpha aminoacide naturel ou d'un dérivé simple de ce dernier, tel un ester méthylique, éthylique, ou un amide primaire dans le cas des acides aminés dicarboxyliques ;
R₁ représente un atome d'hydrogène, un groupement alkyle comprenant 1 à 6 atomes de carbone ou un groupement benzyle ;
et R₂ a la même signification qu'à la revendication 5, à la condition que R₂ soit différent d'un groupe acétyle lorsque A représente un groupe benzyle et R₁ un groupe méthyle.

## Claims (Claims for the following Contracting State(s): AT, BE, CH, LI, DE, DK, FR, GB, IT, LU, NL, SE)

1. A hydroquinone derivative, characterized in that it is constituted by a natural amino acid ester of hydroquinone and in that it has the following formula (I): 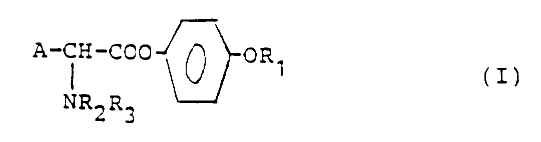 in which:
A corresponds to the radical of a natural alpha-amino acid or a simple derivative thereof, such as a methyl or ethyl ester or a primary amide in the case of dicarboxylic amino acids;
R₁ is a hydrogen atom, an alkyl group containing 1 to 6 carbon atoms or a benzyl group;
R₂ is a hydrogen atom, an acetyl or benzoyl group or an alkylcarbonyl radical with 2 to 20 hydrocarbon units, or else defines a nitrogen-containing ring with A,
with the proviso that R₂ is other than a hydrogen atom when A and R₁ are methyl groups, that R₂ is other than a benzoyl group when A is a hydrogen atom and R₁ is a methyl group, and that R₂ is other than an acetyl group when A is a benzyl group and R₁ is a methyl group; and
R₃ is a hydrogen atom or, only when A and R₂ form a nitrogen-containing ring, an alkylcarbonyl radical with 1 to 20 linear hydrocarbon units or a linear or branched alkyl radical with 1 to 18 hydrocarbon units.

2. A derivative according to Claim 1, characterized in that A is a hydrogen atom, a nitrogen-containing ring in association with R₂, or an alkyl group containing 1 to 4 carbon atoms which is unsubstituted or substituted by one of the following groups: OH, SH, C₆H₄-OH, CO-NH₂, COOH, NH₂, NH-CNH₂-NH₂, C₆H₅, indole, S-CH₃ or imidazole, which substituents may or may not themselves be esterified.

3. A derivative according to Claim 1 or Claim 2, characterized in that A forms, with R₂, products containing a pyrrolidin-2-one ring, A thus defining a pyroglutamic acid radical with R₂, which products have the following formula (II): 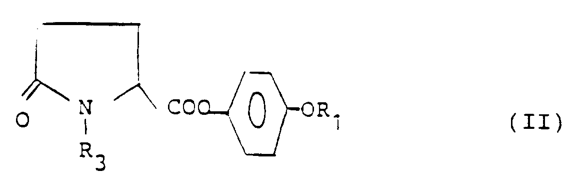 in which R₁ and R₃ are as defined above.

4. A derivative according to any one of Claims 1 to 3, characterized in that it has an amphiphilic character.

5. A derivative according to any one of Claims 1, 2 or 4, characterized in that the radical R₂ is an alkylcarbonyl radical.

6. A derivative according to any one of Claims 1 to 4, characterized in that the radical R₃ is an alkyl group.

7. A derivative according to any one of Claims 1 to 4, characterized in that the radical R₃ is an alkylcarbonyl radical.

8. A pharmaceutical composition, characterized in that it comprises at least a hydroquinone derivative according to any one of claims 1 to 7, in association with at least one pharmaceutically acceptable vehicle.

9. A pharmaceutical composition, useful as depigmenting agent, characterized in that it comprises at least a hydroquinone derivative according to any one of claims 1 to 7.

10. Use of a hydroquinone derivative of formula (I) according to claim 1, which as the following formula (I'): 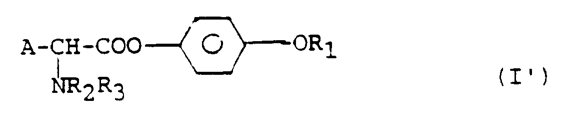 in which A, R₁ and R₃ have the same meaning as above and in which:
- if R₂ is a hydrogen atom, A and R₁ are methyl groups or
- if R₂ is a benzoyl group, A is a hydrogen atom and R₁ is a methyl group or
- if R₂ is an acetyl group, A is a benzyl group and R₁ is a methyl group,
for the preparation of a drug with depigmenting action.

11. Use as cosmetic product of an hydroquinone derivative of formula (I) according to claim 1, which has the following formula (I'): 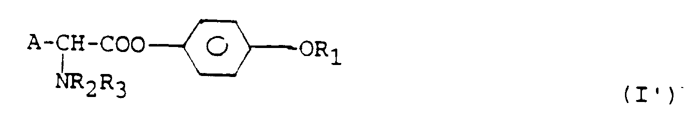 in which A, R₁ and R₃ have the same meaning as above and in which:
- if R₂ is a hydrogen atom, A and R₁ are methyl groups or
- if R₂ is a benzoyl group, A is a hydrogen atom and R₁ is a methyl group or
- if R₂ is an acetyl group, A is a benzyl group and R₁ is a methyl group.

12. A cosmetic composition, characterized in that it comprises at least a product of formula (I) according to any one of claims 1 to 7, if appropriate in association with at least one acceptable vehicle.

13. A composition according to claim 8, claim 9 or claim 12, characterized in that it comprises from 0.01 to 20% by weight of a product of formula (I) according to any one of claims 1 to 7.

14. A composition according to claim 13, characterized in that it contains from 0.1 to 10% by weight of a product of formula (I) according to any one of claims 1 to 7, if appropriate in association with at least one acceptable vehicle.

15. A method of aesthetic treatment for humans to modify the skin's pigmentation, comprising the topical administration of an appropriate amount of a product selected from the group which comprises the products of formula (I) according to any one of claims 1 to 7 and the products of formula (I'): 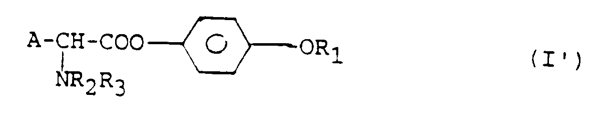 in which A, R₁ and R₃ have the same meaning as above and in which:
- if R₂ is a hydrogen atom, A and R₁ are methyl groups or
- if R₂ is a benzoyl group, A is a hydrogen atom and R₁ is a methyl group or
- if R₂ is an acetyl group, A is a benzyl group and R₁ is a methyl group,
if appropriate in association with at least one acceptable vehicle.

16. The method according to claim 15, characterized in that the product of formula (I) or the product of formula (I') is in the form of a composition comprising 0.01 to 20% by weight of said product.

17. The method according to claim 16, characterized in that said products are in the form of a composition comprising 0.1 to 10% by weight of said products.

18. A method of preparing the derivatives of formula (I) according to Claim 1, characterized in that when R₂ and R₃ are a hydrogen atom, a derivative of the formula p-OH-C₆H₄-OR₁, in which R₁ is as defined above, is reacted with an appropriate amino acid which is protected on the nitrogen and whose carboxylic acid group is activated by a suitable activating reagent, and then in that said protecting group is removed by catalytic hydrogenolysis, carried out in an appropriate inert solvent, to give the compounds of formula (III) below:

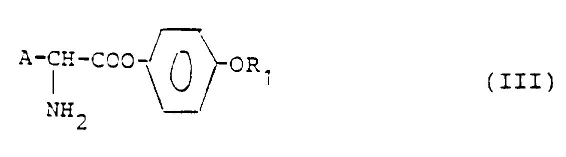

19. A method according to Claim 18, characterized in that the reagents for activating the carboxylic acid group are selected from 1,1-carbonyldiimidazole, 1,1-carbonyldi(1,2,4-triazole), N-hydroxyphthalimide and N-hydroxysuccinimide, preferably 1,1-carbonyldiimidazole.

20. A method of preparing the derivatives according to Claim 5, characterized in that an appropriate amino acid is acylated in a two-phase water/organic solvent medium with a reactive derivative of the acid R₂OH (R₂ being an alkylcarbonyl radical as defined above), preferably the corresponding chloride, to give the compounds of formula (IV):

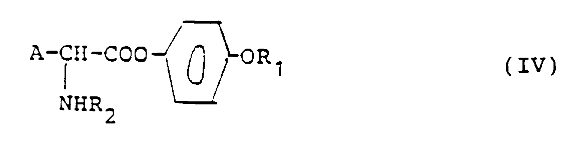

21. A method of preparing the derivatives of formula (II), characterized in that when R₃ is a hydrogen atom, a direct fusion of the corresponding amino acid with the derivative p-OH-C₆H₄OR₁, R₁ being as defined above, is carried out without a solvent and under an inert atmosphere.

22. An intermediate for the preparation of the derivatives of formula I according to Claim 1, characterized in that it has the following formula III: 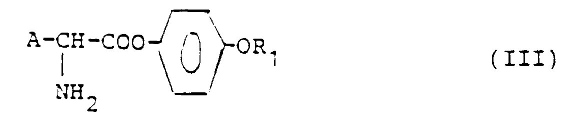 in which:
A corresponds to the radical of a natural alpha-amino acid or a simple derivative thereof, such as a methyl or ethyl ester or a primary amide in the case of dicarboxylic amino acids; and
R₁ is a hydrogen atom, an alkyl group containing 1 to 6 carbon atoms or a benzyl group.

23. An intermediate for the preparation of the derivatives according to Claim 5, characterized in that it has the following formula IV: 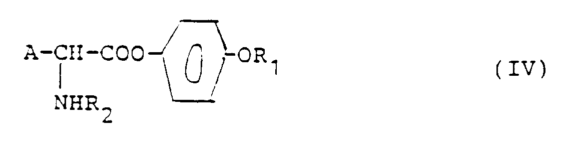 in which:
A corresponds to the radical of a natural alpha-amino acid or a simple derivative thereof, such as a methyl or ethyl ester or a primary amide in the case of dicarboxylic amino acids;
R₁ is a hydrogen atom, an alkyl group containing 1 to 6 carbon atoms or a benzyl group; and
R₂ is as defined in Claim 5, with the proviso that R₂ is other than an acetyl group when A is a benzyl group and R₁ is a methyl group.

## Claims (Claims for the following Contracting State(s): ES, GR)

1. Method of preparing a hydroquinone derivative, characterized in that it comprises for obtaining a natural amino acid ester of hydroquinone, having the following formula (I) : 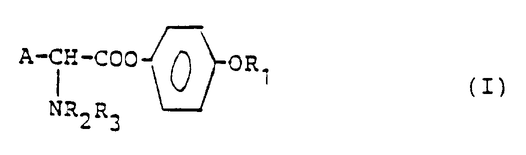 in which :
A corresponds to the radical of a natural alpha-amino acid or a simple derivative thereof, such as a methyl or ethyl ester or a primary amide in the case of dicarboxylic amino acids ;
R₁ is a hydrogen atom, an alkyl group containing 1 to 6 carbon atoms or a benzyl group ;
R₂ is a hydrogen atom, an acetyl or benzoyl group or an alkylcarbonyl radical with 2 to 20 hydrocarbon units, or else defines a nitrogen-containing ring with A,
with the proviso that R₂ is other than a hydrogen atom when A and R₁ are methyl groups, that R₂ is other than a benzoyl group when A is a hydrogen atom and R₁ is a methyl group, and that R₂ is other than an acetyl group when A is a benzyl group and R₁ is a methyl group ; and
R₃ is a hydrogen atom or, only when A and R₂ form a nitrogen-containing ring, an alkylcarbonyl radical with 1 to 20 linear hydrocarbon units or a linear or branched alkyl radical with 1 to 18 hydrocarbon units, the reaction of an appropriate aminoacid and a derivative of the formula p-OH-C₆H₄-OR₁, in which R₁ is as defined above.

2. Method of preparing a derivative according to claim 1, characterized in that A represents a hydrogen atom, a nitrogen-containing ring in association with R₂, or an alkyl group containing 1 to 4 carbon atoms which is unsubstituted or substituted by one of the following groups : OH, SH, C₆H₄-OH, CO-NH₂, COOH, NH₂, NH-CNH₂-NH₂, C₆H₅, indole, S-CH₃, or imidazole, which substituents may or may not themselves be esterified.

3. Process of preparing a derivative according to claim 1 or to claim 2, characterized in that A forms, with R₂, products containing a pyrrolidin-2-one ring, A thus defining a pyroglutamic acid radical with R₂, which products have the following formula (II) : 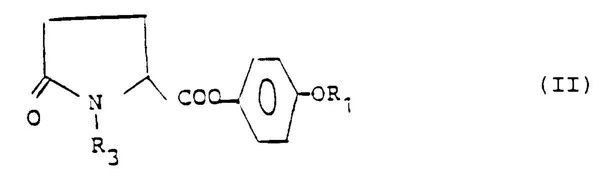 in which R₁ and R₃ are as defined above.

4. Process of preparing a derivative according to any one of claims 1 to 3, characterized in that the obtained product presents an amphiphilic character.

5. Process of preparing a derivative according to any one of claims 1, 2 or 4, characterized in that the radical R₂ is an alkylcarbonyl radical.

6. Process of preparing a derivative according to any one of claims 1 to 4, characterized in that the radical R₃ is an alkyl group.

7. Process of preparing a derivative according to any one of claims 1 to 4, characterized in that the radical R₃ is an alkylcarbonyl radical.

8. Process of preparing a pharmaceutical composition, characterized in that it comprises the incorporation to said composition of at least one hydroquinone derivative obtained by the process according to any one of claims 1 to 7 and at least one pharmaceutically acceptable vehicule.

9. Process of preparing a pharmaceutical composition useful as depigmenting agent, characterized in that it comprises the incorporation to said composition of at least one hydroquinone derivative obtained according to any one of claims 1 to 7.

10. Process of preparing a drug comprising the preparation of a hydroquinone derivative of formula (I), obtained according to claim 1, which have the formula (I'): 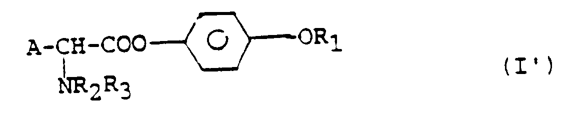 in which A, R₁ and R₃ have the same meaning as above and in which :
- if R₂ is a hydrogen atom, A and R₁ are methyl groups or
- if R₂ is a benzoyl group, A is a hydrogen atom and R₁ is a methyl group or
- if R₂ is an acetyl group, A is a benzyl group and R₁ is a methyl group,
characterized in that the obtained drug is for a depigmenting use.

11. Method of cosmetic treatment comprising the administration of an hydroquinone derivative of formula (I) obtained according to claim 1, having the formula (I') 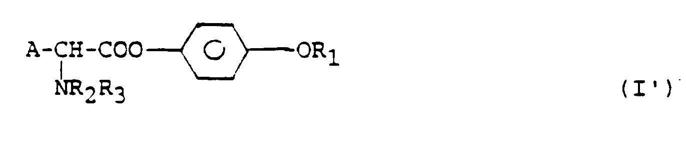 in which A, R₁ and R₃ have the same meaning as above and in which :
- if R₂ is a hydrogen atom, A and R₁ are methyl groups or
- if R₂ is a benzoyl group, A is a hydrogen atom and R₁ is a methyl group or
- if R₂ is an acetyl group, A is a benzyl group and R₁ is a methyl group.

12. Process of preparation of a cosmetic composition, characterized in that it comprises incorporation to said composition of at least one derivative of formula (I) obtained according to any one of claims 1 to 7 and optionally of at least one acceptable vehicule.

13. Process of preparation of a composition obtained according to any one of claims 8, 9 or 12, characterized in that it comprises the incorporation to said composition of 0.01 to 20% by weight of a product of formula (I) obtained according to any one of claims 1 to 7.

14. Process of preparation of composition according to claim 13, characterized in that it comprises the incorporation to said composition of 0.1 to 10% by weight of a product of formula (I) obtained according to any one of claims 1 to 7, and if appropriate at least one acceptable vehicule.

15. A method of aesthetic treatment for humans to modify the skin's pigmentation, comprising the topical administration of an appropriate amount of a product selected from the group which comprises the products of formula (I) according to any one of claims 1 to 7 and the products of formula (I'): 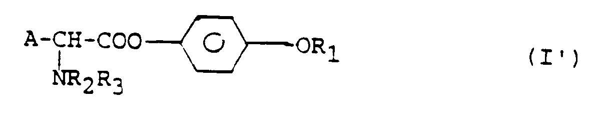 in which A, R₁ and R₃ have the same meaning as above and in which:
- if R₂ is a hydrogen atom, A and R₁ are methyl groups or
- if R₂ is a benzoyl group, A is a hydrogen atom and R₁ is a methyl group or
- if R₂ is an acetyl group, A is a benzyl group and R₁ is a methyl group,
if appropriate in association with at least one acceptable vehicle.

16. The method according to claim 15, characterized in that the product of formula (I) or the product of formula (I') is in the form of a composition comprising 0.01 to 20% by weight of said product.

17. The method according to claim 16, characterized in that said products are in the form of a composition comprising 0.1 to 10% by weight of said products.

18. A method of preparing the derivatives of formula (I) according to Claim 1, characterized in that when R₂ and R, are a hydrogen atom, a derivative of the formula p-OH-C₆H₄-OR₁, in which R₁ is as defined above, is reacted with an appropriate amino acid which is protected on the nitrogen and whose carboxylic acid group is activated by a suitable activating reagent, and then in that said protecting group is removed by catalytic hydrogenolysis, carried out in an appropriate inert solvent, to give the compounds of formula (III) below:

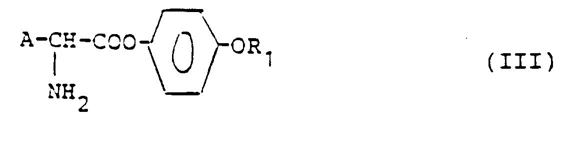

19. A method according to Claim 18, characterized in that the reagents for activating the carboxylic acid group are selected from 1,1-carbonyldiimidazole, 1,1-carbonyldi(1,2,4-triazole), N-hydroxyphthalimide and N-hydroxysuccinimide, preferably 1,1-carbonyldiimidazole.

20. A method of preparing the derivatives according to Claim 5, characterized in that an appropriate amino acid is acylated in a two-phase water/organic solvent medium with a reactive derivative of the acid R₂OH (R₂ being an alkylcarbonyl radical as defined above), preferably the corresponding chloride, to give the compounds of formula (IV):

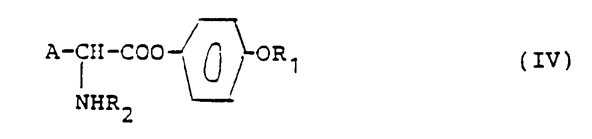

21. A method of preparing the derivatives of formula (II), characterized in that when R₃ is a hydrogen atom, a direct fusion of the corresponding amino acid with she derivative p-OH-C₆H₄OR₁, R₁ being as defined above, is carried out without a solvent and under an inert atmosphere.

22. An intermediate for the preparation of the derivatives of formula I according to Claim 1, characterized in that it has the following formula III: 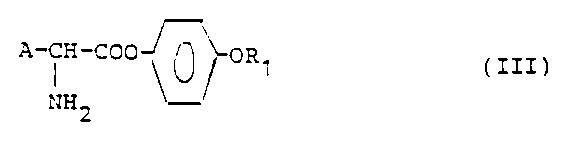 in which:
A corresponds to the radical of a natural alpha-amino acid or a simple derivative thereof, such as a methyl or ethyl ester or a primary amide in the case of dicarboxylic amino acids; and
R₁ is a hydrogen atom, an alkyl group containing 1 to 6 carbon atoms or a benzyl group.

23. An intermediate for the preparation of the derivatives according to Claim 5, characterized in that it has the following formula IV: 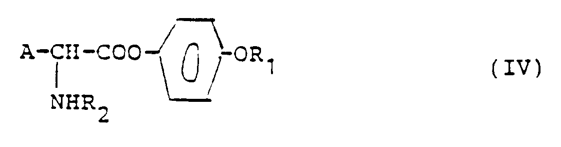 in which:
A corresponds to the radical of a natural alpha-amino acid or a simple derivative thereof, such as a methyl or ethyl ester or a primary amide in the case of dicarboxylic amino acids;
R₁ is a hydrogen atom, an alkyl group containing 1 to 6 carbon atoms or a benzyl group; and
R₂ is as defined in Claim 5, with the proviso that R₂ is other than an acetyl group when A is a benzyl group and R₁ is a methyl group.

## Patentansprüche (Patentansprüche für folgende(n) Vertragsstaat(en): AT, BE, CH, LI, DE, DK, FR, GB, IT, LU, NL, SE)

1. Hydrochinonderivat, dadurch **gekennzeichnet,** daß es aus einem Hydrochinonester einer natürlichen Aminosäure besteht und daß es der folgenden Formel (I) entspricht 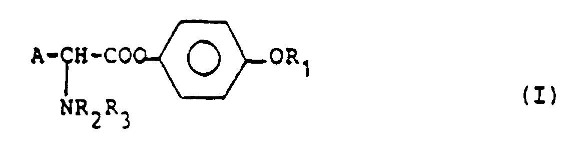 worin,
A für einen Rest einer natürlichen alpha-Aminosäure oder für ein einfaches Derivat dieser letzteren, wie einen Methylester, Ethylester, oder ein primäres Amid im Fall der Aminodicarbonsäuren steht;
R₁ ein Wasserstoffatom, eine Alkylgruppe, umfassend 1 bis 6 Kohlenstoffatome, oder eine Benzylgruppe bedeutet;
R₂ ein Wasserstoffatom, eine Acetyl- oder Benzoylgruppe, einen Alkylcarbonylrest mit einer 2 bis 20gliedrigen Kohlenwasserstoffkette bedeutet oder zusammen mit A für einen stickstoffhaltigen Ring steht,
mit der Maßgabe, daß R₂ nicht für ein Wasserstoffatom steht, wenn A oder R₁ Methylgruppen bedeuten, daß R₂ nicht für eine Benzoylgruppe steht, wenn A ein Wasserstoffatom und R₁ eine Methylgruppe bedeutet und daß R₂ nicht für eine Acetylgruppe steht, wenn A eine Benzylgruppe und R₁ eine Methylgruppe bedeuten;
R₃ für ein Wasserstoffatom und, nur wenn A und R₂ einen Stickstoff-enthaltenden Ring bedeuten, für einen Alkylcarbonylrest mit einer 1 bis 20gliedrigen linearen Kohlenwasserstoffkette oder für einen linearen oder verzweigten Alkylrest mit 1 bis 18gliedriger Kohlenwasserstoffkette steht.

2. Derivat nach Anspruch 1, dadurch **gekennzeichnet,** daß A für ein Wasserstoffatom, zusammen mit R₂ für einen Stickstoff-enthaltenden Ring, oder für eine 1 bis 4 Kohlenstoffatome umfassende Alkylgruppe, gegebenenfalls substituiert mit einer der folgenden Gruppen, OH, SH, C₆H₄-OH, CO-NH₂, COOH, NH₂, NH-CNH₂-NH₂, C₆H₅, Indol, S-CH₃, Imidazol, wobei die Substituenten gegebenenfalls ihrerseits verestert sein können, steht.

3. Derivat nach Anspruch 1 oder 2, dadurch **gekennzeichnet,** daß A mit R₂ Produkte, umfassend einen Pyrrolidin-2-on-Ring, bestimmt, wobei A auf diese Weise mit R₂ einen Pyroglutaminsäurerest definiert und wobei diese Produkte der folgenden Formel (II) entsprechen 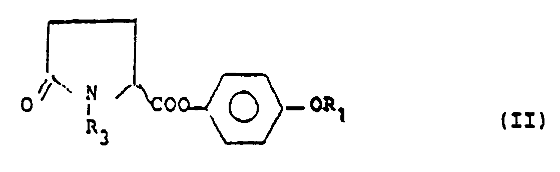 worin
R₁ und R₃ dieselben Bedeutungen wie oben angegeben haben.

4. Derivat nach einem der Ansprüche 1 bis 3, dadurch **gekennzeichnet,** daß es einen amphiphilen Charakter aufweist.

5. Derivat nach einem der Ansprüche 1, 2 oder 4, dadurch **gekennzeichnet,** daß der Rest R₂ für einen Alkylcarbonylrest steht.

6. Derivat nach einem der Ansprüche 1 bis 4, dadurch **gekennzeichnet,** daß der Rest R₃ für eine Alkylgruppe steht.

7. Derivat nach einem der Ansprüche 1 bis 4, dadurch **gekennzeichnet,** daß der Rest R₃ für einen Alkylcarbonylrest steht.

8. Pharmazeutisches Präparat, dadurch **gekennzeichnet,** daß es mindestens ein Hydrochinonderivat nach einem der Ansprüche 1 bis 7 zusammen mit mindestens einem pharmazeutisch annehmbaren Vehikel umfaßt.

9. Pharmazeutisches Präparat, geeignet als Depigmentierungsmittel, dadurch **gekennzeichnet,** daß es mindestens ein Hydrochinonderivat nach einem der Ansprüche 1 bis 7 enthält.

10. Verwendung eines Hydrochinonderivats der Formel (I) gemäß Anspruch 1, das der Formel (I') entspricht 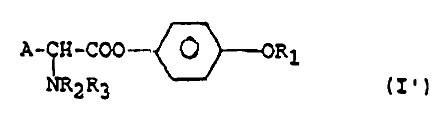 worin A, R₁ und R₃ dieselbe Bedeutung wie oben angegeben haben und worin,
wenn R₂ ein Wasserstoffatom bedeutet, A und R₁ für Methylgruppen stehen oder,
wenn R₂ eine Benzoylgruppe bedeutet, A für ein Wasserstoffatom und R₁ für eine Methylgruppe stehen oder,
wenn R₂ eine Acetylgruppe bedeutet, A für eine Benzylgruppe und R₁ für eine Methylgruppe stehen, um ein Medikament zu erhalten, das für eine Verwendung als Depigmentierungsmittel bestimmt ist.

11. Verwendung als Kosmetikprodukt eines Hydrochinderivats der Formel (I) gemäß Anspruch 1, das der Formel (I') entspricht 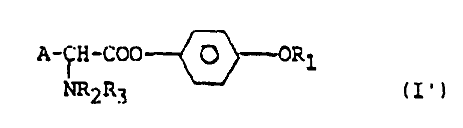 worin A, R₁ und R₃ dieselbe Bedeutung wie oben angegeben haben und worin,
wenn R₂ ein Wasserstoffatom bedeutet, A und R₁ für Methylgruppen stehen oder,
wenn R₂ eine Benzoylgruppe bedeutet, A für ein Wasserstoffatom und R₁ für eine Methylgruppe stehen oder,
wenn R₂ eine Acetylgruppe bedeutet, A für eine Benzylgruppe und R₁ für eine Methylgruppe stehen.

12. Kosmetisches Präparat, dadurch **gekennzeichnet,** daß es mindestens ein Produkt nach Formel (I) gemäß einem der Ansprüche 1 bis 7, gegebenenfalls zusammen mit mindestens einem annehmbaren Vehikel, umfaßt.

13. Präparat nach einem der Ansprüche 8, 9 oder 12, dadurch **gekennzeichnet**, daß es 0,01 bis 20 Gew.-% eines Produkts der Formel (I) gemäß einem der Ansprüche 1 bis 7 umfaßt.

14. Präparat nach Anspruch 13, dadurch **gekennzeichnet,** daß es 0,1 bis 10 Gew.-% eines Produkts der Formel (I) gemäß einem der Ansprüche 1 bis 7 enthält, gegebenenfalls zusammen mit mindestens einem annehmbaren Vehikel.

15. Verfahren zur kosmetischen Behandlung des Menschen, um die Hautpigmentierung zu modifizieren, **gekennzeichnet** durch die topische Applikation einer geeigneten Menge eines Produkts, ausgewählt aus der Gruppe bestehend aus Produkten der Formel (I) gemäß einem der Ansprüche 1 bis 7 und den Produkten der Formel (I') 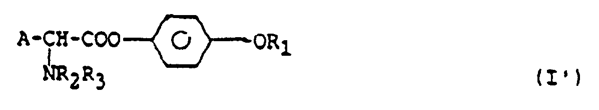 worin A, R₁ und R₃ dieselbe Bedeutung wie oben angegeben haben und worin,
wenn R₂ ein Wasserstoffatom bedeutet, A und R₁ für Methylgruppen stehen oder,
wenn R₂ eine Benzoylgruppe bedeutet, A für ein Wasserstoffatom und R₁ für eine Methylgruppe stehen oder,
wenn R₂ eine Acetylgruppe bedeutet, A für eine Benzylgruppe und R₁ für eine Methylgruppe stehen,
gegebenenfalls zusammen mit mindestens einem akzeptablen Vehikel.

16. Verfahren nach Anspruch 15, dadurch **gekennzeichnet,** daß das Produkt der Formel (I) oder das Produkt der Formel (I') in Form eines Präparats, umfassend 0,01 bis 20 Gew.-% dieses Produkts, vorliegt.

17. Verfahren nach Anspruch 16, dadurch **gekennzeichnet,** daß diese Produkte in Form eines Präparats, umfassend 0,1 bis 10 Gew.-% dieser Produkte, vorliegen.

18. Verfahren zur Herstellung von Derivaten nach Formel (I) gemäß Anspruch 1, dadurch **gekennzeichnet,** daß man, wenn R₂ und R₃ Wasserstoffatome bedeuten, ein Derivat der Formel p-OH-C₆H₄-OR₁, worin R₁ dieselbe Bedeutung wie oben angegeben hat, mit einer am Stickstoff geschützten geeigneten Aminosäure, deren Carbonsäurefunktion durch ein geeignetes Aktivierungsreagens aktiviert worden ist, umsetzt, daß man dann die Schutzgruppe durch katalytische Hydrogenolyse, durchgeführt in einem geeigneten inerten Lösungsmittel, eliminiert, um Verbindungen der folgenden Formel (III) zu erhalten

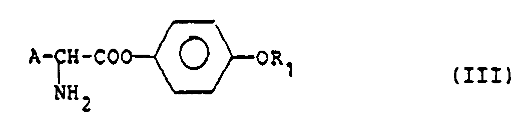

19. Verfahren nach Anspruch 18, dadurch **gekennzeichnet,** daß die Aktivierungsreagentien für die Carboxylfunktion aus 1,1-Carbonyldiimidazol, 1,1-Carbonyldi(1,2,4-triazol), N-Hydroxyphthalimid, N-Hydroxysuccinimid ausgewählt werden, wobei 1,1-Carbonyldiimidazol bevorzugt ist.

20. Verfahren zur Herstellung von Derivaten nach Anspruch 5, dadurch **gekennzeichnet,** daß man die Acylierung einer geeigneten Aminosäure in einem Zwei-Phasen-Milieu Wasser/organisches Lösungsmittel mit einem reaktiven Derivat der Säure R₂OH (R₂ steht für einen Alkylcarbonylrest wie oben definiert), vorzugsweise das entsprechende Chlorid, durchführt, um die Verbindungen der Formel (IV) zu erhalten

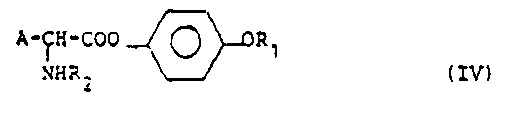

21. Verfahren zur Herstellung von Derivaten der Formel (II), dadurch **gekennzeichnet,** daß man, wenn R₃ für ein Wasserstoffatom steht, eine direkte Verschmelzung ohne Lösungsmittel und unter inerter Atmosphäre der entsprechenden Aminosäure mit dem Derivat p-OH-C₆H₄-OR₁ durchführt, wobei R₁ dieselbe Bedeutung wie oben angegeben hat.

22. Zwischenprodukt für die Herstellung von Derivaten der Formel (I) nach Anspruch 1, dadurch **gekennzeichnet,** daß es der folgenden Formel (III) entspricht 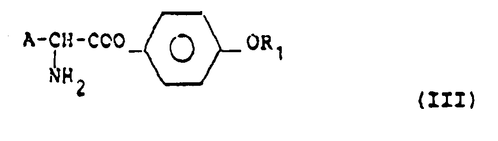 worin
A für einen Rest einer natürlichen alpha-Aminosäure oder eines einfachen Derivats der letzteren, wie einen Methylester, Ethylester, oder ein primäres Amid im Fall der Aminodicarbonsäuren steht,
R₁ für ein Wasserstoffatom, eine Alkylgruppe umfassend 1 bis 6 Kohlenstoffatome oder eine Benzylgruppe steht.

23. Zwischenprodukt für die Herstellung der Derivate gemäß Anspruch 5, dadurch **gekennzeichnet,** daß es der folgenden Formel (IV) entspricht 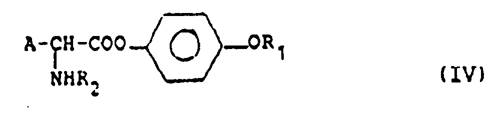 worin
A für einen Rest einer natürlichen alpha-Aminosäure oder eines einfachen Derivats der letzteren, wie einen Methylester, Ethylester, oder ein primäres Amid im Fall der Aminodicarbonsäuren steht,
R₁ für ein Wasserstoffatom, eine Alkylgruppe umfassend 1 bis 6 Kohlenstoffatome oder eine Benzylgruppe steht, und
R₂ dieselbe Bedeutung wie in Anspruch 5 hat, mit der Maßgabe, daß R₂ keine Acetylgruppe bedeutet, wenn A für eine Benzylgruppe und R₁ für eine Methylgruppe stehen.

## Patentansprüche (Patentansprüche für folgende(n) Vertragsstaat(en): ES, GR)

1. Verfahren zur Herstellung eines Hydrochinonderivats, dadurch **gekennzeichnet,** daß es für die Herstellung eines Hydrochinonesters einer natürlichen Aminosäure, gemäß der folgenden Formel (I) 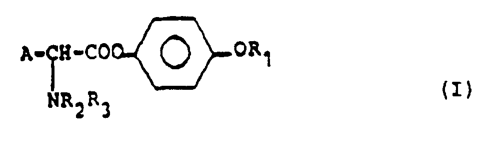 worin
A für einen Rest einer natürlichen alpha-Aminosäure oder für ein einfaches Derivat dieser letzteren, wie einen Methylester, Ethylester, oder ein primäres Amid im Fall der Aminodicarbonsäuren steht;
R₁ ein Wasserstoffatom, eine Alkylgruppe, umfassend 1 bis 6 Kohlenstoffatome, oder eine Benzylgruppe bedeutet;
R₂ ein Wasserstoffatom, eine Acetyl- oder Benzoylgruppe,
einen Alkylcarbonylrest mit einer 2 bis 20gliedrigen Kohlenwasserstoffkette bedeutet oder zusammen mit A für einen stickstoffhaltigen Ring steht,
mit der Maßgabe, daß R₂ nicht für ein Wasserstoffatom steht, wenn A oder R₁ Methylgruppen bedeuten, daß R₂ nicht für eine Benzoylgruppe steht, wenn A ein Wasserstoffatom und R₁ eine Methylgruppe bedeutet und daß R₂ nicht für eine Acetylgruppe steht, wenn A eine Benzylgruppe und R₁ eine Methylgruppe bedeuten;
R₃ für ein Wasserstoffatom und, nur wenn A und R₂ einen Stickstoff-enthaltenden Ring bedeuten, für einen Alkylcarbonylrest mit einer 1 bis 20gliedrigen linearen Kohlenwasserstoffkette oder für einen linearen oder verzweigten Alkylrest mit 1 bis 18gliedriger Kohlenwasserstoffkette steht,
die Reaktion einer Aminosäure und eines Derivats p-OH-C₆H₄-OR₁, worin R₁ dieselbe Bedeutung wie oben angegeben hat, umfaßt.

2. Verfahren zur Herstellung eines Derivats nach Anspruch 1, dadurch **gekennzeichnet,** daß A für ein Wasserstoffatom, zusammen mit R₂ für einen Stickstoff-enthaltenden Ring, oder für eine 1 bis 4 Kohlenstoffatome umfassende Alkylgruppe, gegebenenfalls substituiert mit einer der folgenden Gruppen, OH, SH, C₆H₄-OH, CO-NH₂, COOH, NH₂, NH-CNH₂-NH₂, C₆H₅, Indol, S-CH₃, Imidazol, wobei die Substituenten gegebenenfalls ihrerseits verestert sein können, steht.

3. Verfahren zur Herstellung eines Derivats nach Anspruch 1 oder 2, dadurch **gekennzeichnet,** daß A mit R₂ Produkte, umfassend einen Pyrrolidin-2-on-Ring bestimmt, wobei A auf diese Weise mit R₂ einen Pyroglutaminsäurerest definiert und wobei diese Produkte der folgenden Formel (II) entsprechen 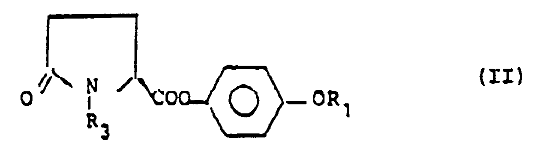 worin
R₁ und R₃ diesselben Bedeutungen wie oben angegeben haben.

4. Verfahren zur Herstellung eines Derivats nach einem der Ansprüche 1 bis 3, dadurch **gekennzeichnet,** daß das erhaltene Produkt einen amphiphilen Charakter aufweist.

5. Verfahren zur Herstellung eines Derivats nach einem der Ansprüche 1, 2 oder 4, dadurch **gekennzeichnet**, daß der Rest R₂ für einen Alkylcarbonylrest steht.

6. Verfahren zur Herstellung eines Derivats nach einem der Ansprüche 1 bis 4, dadurch **gekennzeichnet,** daß der Rest R₃ für eine Alkylgruppe steht.

7. Verfahren zur Herstellung eines Derivats nach einem der Ansprüche 1 bis 4, dadurch **gekennzeichnet,** daß der Rest R₃ für einen Alkylcarbonylrest steht.

8. Verfahren zur Herstellung eines pharmazeutischen Präparats, dadurch **gekennzeichnet,** daß es das Einarbeiten von mindestens einem Hydrochinonderivat, erhalten nach einem der Ansprüche 1 bis 7, und von mindestens einem pharmazeutisch annehmbaren Vehikel in das Präparat umfaßt.

9. Verfahren zur Herstellung eines pharmazeutischen Präparats, das als Depigmentierungsmittel nützlich ist, dadurch **gekennzeichnet,** daß es das Einarbeiten von mindestens einem Hydrochinonderivat, erhalten nach einem der Ansprüche 1 bis 7, in das Präparat umfaßt.

10. Verfahren zur Herstellung eines Medikaments, umfassend die Herstellung eines Hydrochinonderivats der Formel (I), erhalten nach Anspruch 1, das der Formel (I') entspricht 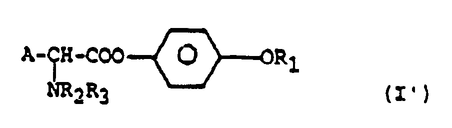 worin A, R₁, R₂ und R₃ dieselbe Bedeutung wie oben angegeben haben und worin,
wenn R₂ ein Wasserstoffatom bedeutet, A und R₁ für Methylgruppen stehen oder,
wenn R₂ eine Benzoylgruppe bedeutet, A für ein Wasserstoffatom und R₁ für eine Methylgruppe stehen oder,
wenn R₂ eine Acetylgruppe bedeutet, A für eine Benzylgruppe und R₁ für eine Methylgruppe stehen,
dadurch **gekennzeichnet,** daß das erhaltene Medikament zur Verwendung als Depigmentierungsmittel bestimmt ist.

11. Verfahren zur kosmetischen Behandlung, umfassend die Applikation eines Hydrochinderivats der Formel (I), erhalten nach Anspruch 1, das der Formel (I') entspricht 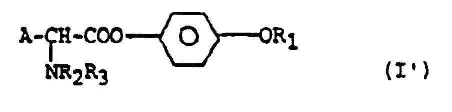 worin A, R₁, R₂ und R₃ dieselbe Bedeutung wie oben angegeben haben und worin,
wenn R₂ ein Wasserstoffatom bedeutet, A und R₁ für Methylgruppen stehen oder,
wenn R₂ eine Benzoylgruppe bedeutet, A für ein Wasserstoffatom und R₁ für eine Methylgruppe stehen oder,
wenn R₂ eine Acetylgruppe bedeutet, A für eine Benzylgruppe und R₁ für eine Methylgruppe stehen.

12. Verfahren zur Herstellung eines kosmetischen Präparats, dadurch **gekennzeichnet,** daß es das Einarbeiten von mindestens einem Produkt nach Formel (I), erhalten nach einem der Ansprüche 1 bis 7, und gegebenenfalls eines geeigneten Vehikels umfaßt.

13. Verfahren zur Herstellung eines Präparats, erhalten nach einem der Ansprüche 8, 9 oder 12, dadurch **gekennzeichnet,** daß es das Einarbeiten von 0,01 bis 20 Gew.-% eines Produkts der Formel (I), erhalten nach einem der Ansprüche 1 bis 7, in das Präparat umfaßt.

14. Verfahren zur Herstellung eines Präparats nach Anspruch 13, dadurch **gekennzeichnet,** daß es das Einarbeiten von 0,1 bis 10 Gew.-% eines Produkts der Formel (I), erhalten nach einem der Ansprüche 1 bis 7, und gegebenenfalls die Einarbeitung von mindestens einem annehmbaren Vehikel umfaßt.

15. Verfahren zur kosmetischen Behandlung des Menschen, um die Hautpigmentierung zu modifizieren, **gekennzeichnet** durch die topische Applikation einer geeigneten Menge eines Produkts, ausgewählt aus der Gruppe bestehend aus Produkten der Formel I, gemäß einem der Ansprüche 1 bis 7 und den Produkten der Formel (I') 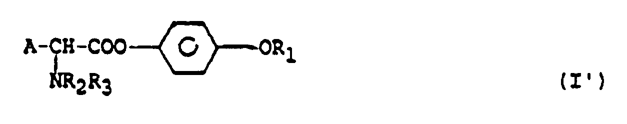 worin A, R₁, R₂ und R₃ dieselbe Bedeutung wie oben angegeben haben und worin,
wenn R₂ ein Wasserstoffatom bedeutet, A und R₁ für Methylgruppen stehen oder,
wenn R₂ eine Benzoylgruppe bedeutet, A für ein Wasserstoffatom und R₁ für eine Methylgruppe stehen oder,
wenn R₂ eine Acetylgruppe bedeutet, A für eine Benzylgruppe und R₁ für eine Methylgruppe stehen,
gegebenenfalls in Verbindung mit mindestens einem akzeptablen Vehikel.

16. Verfahren nach Anspruch 15, dadurch **gekennzeichnet,** daß das Produkt der Formel (I) oder das Produkt der Formel (I') in Form eines Präparats, umfassend 0,01 bis 20 Gew.-% dieses Produkts, vorliegt.

17. Verfahren nach Anspruch 16, dadurch **gekennzeichnet,** daß diese Produkte in Form eines Präparats, umfassend 0,1 bis 10 Gew.-% dieser Produkte, vorliegen.

18. Verfahren zur Herstellung von Derivaten nach Formel (I) gemäß Anspruch 1, dadurch **gekennzeichnet,** daß man, wenn R₂ und R₃ Wasserstoffatome bedeuten, ein Derivat der Formel p-OH-C₆H₄-OR₁, worin R₁ dieselbe Bedeutung wie oben angegeben hat, mit einer am Stickstoff geschützten geeigneten Aminosäure, deren Carbonsäurefunktion durch ein geeignetes Aktivierungsreagens aktiviert worden ist, umsetzt, daß man dann die Schutzgruppe durch katalytische Hydrogenolyse, durchgeführt in einem geeigneten inerten Lösungsmittel, eliminiert, um Verbindungen der folgenden Formel (III) zu erhalten

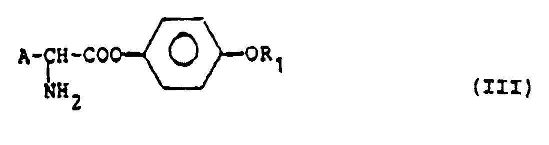

19. Verfahren nach Anspruch 18, dadurch **gekennzeichnet,** daß die Aktivierungsreagentien für die Carboxylfunktion aus 1,1-Carbonyldiimidazol, 1,1-Carbonyldi(1,2,4-triazol), N-Hydroxyphthalimid, N-Hydroxysuccinimid ausgewählt werden, wobei 1,1-Carbonyldiimidazol bevorzugt ist.

20. Verfahren zur Herstellung von Derivaten nach Anspruch 5, dadurch **gekennzeichnet,** daß man die Acylierung einer geeigneten Aminosäure in einem Zwei-Phasen-Milieu Wasser/organisches Lösungsmittel mit einem reaktiven Derivat der Säure R₂OH (R₂ steht für einen Alkylcarbonylrest wie oben definiert), vorzugsweise das entsprechende Chlorid, durchführt, um die Verbindungen der Formel (IV) zu erhalten

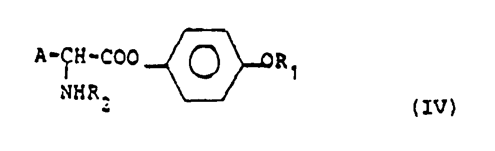

21. Verfahren zur Herstellung von Derivaten der Formel (II), dadurch **gekennzeichnet,** daß man, wenn R₃ für ein Wasserstoffatom steht, eine direkte Verschmelzung ohne Lösungsmittel und unter inerter Atmosphäre der entsprechenden Aminosäure mit dem Derivat p-OH-C₆H₄-OR₁ durchführt, wobei R₁ dieselbe Bedeutung wie oben angegeben hat.

22. Zwischenprodukt für die Herstellung von Derivaten der Formel (I) nach Anspruch 1, dadurch **gekennzeichnet,** daß es der folgenden Formel (III) 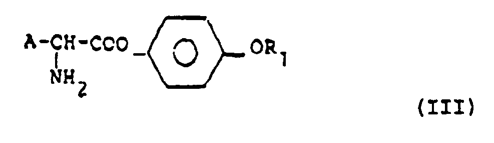 entspricht, worin
A für einen Rest einer natürlichen alpha-Aminosäure oder eines einfachen Derivats der letzteren, wie einen Methylester, Ethylester oder ein primäres Amid im Fall der Aminodicarbonsäuren steht,
R₁ für ein Wasserstoffatom, eine Alkylgruppe, umfassend 1 bis 6 Kohlenstoffatome, oder eine Benzylgruppe steht.

23. Zwischenprodukt für die Herstellung der Derivate gemäß Anspruch 5, dadurch **gekennzeichnet,** daß es der folgenden Formel (IV) entspricht 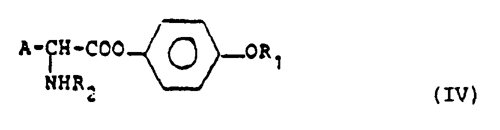 worin
A für einen Rest einer natürlichen alpha-Aminosäure oder eines einfachen Derivats der letzteren, wie einen Methylester, Ethylester, oder ein primäres Amid im Fall der Aminodicarbonsäuren steht,
R₁ für ein Wasserstoffatom, eine Alkylgruppe, umfassend 1 bis 6 Kohlenstoffatome, oder eine Benzylgruppe steht, und
R₂ dieselbe Bedeutung wie in Anspruch 5 hat, mit der Maßgabe, daß R₂ keine Acetylgruppe bedeutet, wenn A für eine Benzylgruppe und R₁ für eine Methylgruppe stehen.
